# EUROPEAN PATENT SPECIFICATION

(11) **EP 3 893 918 B1**
(45) Date of publication and mention of the grant of the patent: **25.09.2024**
(21) Application number: 19827645.3
(22) Date of filing: 10.12.2019
(51) Int. Cl.: A61K 38/22, A61P 27/06, A61P 27/02, A61K 9/00, A61K 9/107, A61K 9/51

(54) **VASODILATORS FOR USE IN THE TREATMENT OF A RETINAL ISCHEMIC DISORDER**
VASODILATOREN ZUR VERWENDUNG BEI DER BEHANDLUNG EINER ISCHÄMISCHEN ERKRANKUNG DER NETZHAUT
VASODILATATEURS DESTINÉS À ÊTRE UTILISÉS DANS LE TRAITEMENT D'UN TROUBLE ISCHÉMIQUE RÉTINIEN

(30) Priority: 10.12.2018 DK PA201800978
(43) Date of publication of application: 20.10.2021
(73) Proprietor: Rigshospitalet, 2100 Copenhagen Ø (DK)
(72) Inventor: HAANES, Kristian, Agmund, 2600 Glostrup (DK); FEDULOV, Vadim, 2600 Glostrup (DK); EDVINSSON, Lars, 2600 Glostrup (DK); WARFVINGE, Karin, 2600 Glostrup (DK)
(74) Representative: Nex & Phister Law & IP Aps
(86) International application number: PCT/EP2019/084442
(87) International publication number: WO 2020/120480

(56) References cited:
- WO-A1-2018/045083
- DE-A1- 3 540 149
- US-A1- 2007 260 203
- US-A1- 2011 130 334
- SAKAMOTO KENJI ET AL: "Activation of the TRPV1 channel attenuates N-methyl-d-aspartic acid-induced neuronal injury in the rat retina", EUROPEAN JOURNAL OF PHARMACOLOGY, ELSEVIER SCIENCE, NL, vol. 733, 1 April 2014 (2014-04-01), pages 13 - 22, XP028663903, ISSN: 0014-2999, DOI: 10.1016/J.EJPHAR.2014.03.035
- AKIRA IMAI ET AL: "Adrenomedullin Suppresses Vascular Endothelial Growth Factor-Induced Vascular Hyperpermeability and Inflammation in Retinopathy", AMERICAN JOURNAL OF PATHOLOGY., vol. 187, no. 5, 1 May 2017 (2017-05-01), US, pages 999 - 1015, XP055676110, ISSN: 0002-9440, DOI: 10.1016/j.ajpath.2017.01.014
- DATABASE EMBASE [online] ELSEVIER SCIENCE PUBLISHERS, AMSTERDAM, NL; 1 September 2018 (2018-09-01), HAANES K A ET AL: "Effects of calcitonin gene-related peptide on the arterial vasodilation in the eye and implications for retinal function", XP002798294, Database accession no. EMB-624305672
- HAANES K A ET AL: "Effects of calcitonin gene-related peptide on the arterial vasodilation in the eye and implications for retinal function", CEPHALALGIA 20180901 SAGE PUBLICATIONS LTD NLD, vol. 38, no. Supplement 1, 1 September 2018 (2018-09-01), pages 32 CONF 20180906 to 20180909 London - 17th Bien, ISSN: 1468-2982

## Description

### FIELD

The present invention is aimed at addressing the root-cause of the majority of ischemic eye diseases by stopping the progressive degeneration of the retina through reversal of constriction of vasculature in the eye that leads to ischemic damage. The present invention discloses vasodilators, in particular CGRP peptides, in topical/intravitreal solutions as well as adenoviral-mediated gene therapy that expresses the peptides as a novel treatment for ischemic eye diseases such as Glaucoma and Diabetic Retinopathy that currently have very limited disease halting therapeutic options.

### BACKGROUND

In several major eye diseases such as Glaucoma and Diabetic Retinopathy, one of the main causes of damage is upregulation of vasoconstrictive peptides such as Endothelin-1 that leads to reduced blood flow to the eye and results in neuro-degeneration of the retina.

Current treatment cannot stop these often chronic and progressive degenerations. Several treatment options that are currently on the market are aimed at merely suppressing some of the symptoms of the disease, without the ability of stopping the progression of disease that leads to e.g. blindness.

The neuropeptide Calcitonin gene-related peptide (CGRP) has long been postulated to play an integral role in the pathophysiology of migraine, to protect against ischemia/reperfusion injury in the liver, and CGRP is known to be a highly potent vasoactive peptide released from sensory nerves, which is now proposed to have protective effects in several systemic cardiovascular diseases.

SAKAMOTO, K. et al.: "Activation of the TRPV1 channel attenuates N-methyl-d-aspartic acid-induced neuronal injury in the rat retina", EUROPEAN JOURNAL OF PHARMACOLOGY, 2014, Vol. 733, pages 13-22 applies intravitreal CGRP injections, however they only monitor the effect of CGRP on intravitreal NMDA induced neuronal damage, which is not a retinal ischemic disorder in a mammal.

US 2006/025386 relates to the use of diazoxide (7-chloro-3methyl-2H1-,2,4-benzothiadiazine-1,1-dioxide) in the treatment of retinal ischemia. Diazoxide acts as a positive allosteric modulator of the AMPA and kainate receptors,

Asami Mori et al.: Vasodilator Effects of Elcatonin, a Synthetic Eel Calcitonin, on Retinal Blood Vessels in Rats. Biol. Pharm. Bull.38, 1536-1541(2015), examine the effects of Elcatonin, on rat retinal blood vessels to determine how diabetes affects the retinal vascular responses. The results presented suggest that Elcatonin dilates rat retinal blood vessels via NO-and COX-dependent mechanisms, and that the adenylyl cyclase-adenosine 3', 5'-cyclic monophosphate system plays a major role in the vasodilator mechanisms. The retinal vasodilatory effects of Elcatonin seem to be preserved at early stages of diabetes. However, the detailed mechanism of the vasodilator response to Elcatonin remains unclear and may stimulate tumor angiogenesis through the calcitonin receptor.

Gene transfer is also used for retinal disease, for example in WO13024433, where a therapeutic nucleic acid of interest inserted into a plasmid is transferred to the eye by electroporation.

WO 2018/045083 discloses calcitonin gene-related peptide (CORP) receptor antagonist for use for treating nerve injury including glaucoma.

Akira Imai et at., investigated the effect of adrenomedullin (ADM) on diabetic macular edema (DME) in Kimba mice, a model of VEGF-induced retinopathy. Intravitreal or systemic administration of ADM to Kimba mice ameliorated both the capillary dropout and vascular leakage. The administration of VEGF enhanced vascular permeability, but the co-administration of ADM suppressed the effect, in part by enhancing tight junction formation between endothelial cells. In addition, a comprehensive PCR array analysis showed that ADM administration suppressed various molecules related to inflammation and NF-kB signaling within retinas. The adrenomedullin formulated for intravitreal administration is considered ot represent "an ocular drug delivery system".

DE 35 40 149 A1 use of-1 (benztriazol-4-yloxy) 3- [2- (2-methoxyphenoxy) ethylamino] -2-propanol as antiglaucomers, pharmaceuticals containing these substances, the optically active R and S isomers Of this substance as well as methods for producing the same.

US 2007/260203 disclosed a method of improving or maintaining vision of an eye of a patient, comprising the step of placing a biodegradable intraocular implant ni an eye of the patient, the implant comprising a therapeutic agent which is a vasodilator wherein the implant degrades at a rate effective to sustain release of an amount of the therapeutic agent from the implant effective to improve or maintain vision in the eye of the patient. Exemplified compounds are bosentan, vardenafil or pilocarpine.

US 2011/130334 disclose a method of treating reperfusion injury comprising administering a composition comprising a ß2-adrenergic receptor agonist and a vasodilator in an amount sufficient to reduce reperfusion injury to a tissue, wherein the vasodilator is calcitonin gene-related peptide (CGRP), adrenomedullin, or amylin. Reperfusion injury can result from many causes, including retinal ischemia.

HAANES KAET AL: The in vivo effect of subconjunctival CGRP on the ciliary arteries was investigated using fundus imaging in male Sprague Dawley rats. CGRP was shown to be a strong vasodilator in vitro on the porcine ophthalmic artery (Emax dilation 83 $ 5 %), rat ophthalmic artery (Emax dilation 68 t 6 %) and porcine retinal arteries CGRP (Emax dilation 29 $ 9 %). Furthermore, CGRP caused a rapid and large vasodilation in vivo of the rat ciliary arteries following subconjunctival application. Several ischemic eye disorders result from vasoconstriction of vasculature ni the eye, and application of exogenous CGRP in these patients could be used as a treatment to improve function.

However, not all potential vasodilators provide a therapeutic effect locally in the eye. Thus, there remains an unmet need for a therapeutic treatment that can prevent for example blindness from a variety of ischemia-related eye diseases. Currently, there are no available therapeutics to halt for example the progression of Glaucoma and Diabetic Retinopathy. There is limited symptomatic treatment, which does not target or treat the underlying disease locally in the eye.

### SUMMARY

The present invention has a great advantage, as it will be the first to provide a therapeutic compound for full reversal or prevention of ischemic retinal disorders by means of applying a vasodilator locally via ocular drug delivery systems comprising a vasodilator direct to the ischemic incident. It is therefore an object of the present invention to provide a medicament capable of treating ischemic eye disease.

In its broadest aspect, the present invention relates to a vasodilator for use in the treatment of a retinal ischemic disorder in a mammal. The vasodilator acts locally via an ocular drug delivery system. The process is the opposite of vasoconstriction, as vasodilation is the widening of blood vessels.

The present invention provides therapeutic compounds that can be applied potentially as an eye drop formulation, for injection preparation, or most valuably for being used as gene therapy for curing of the ischemic retinal disorder. In particular, Adenoviral-mediated gene therapy expressing a peptide having vasodilatory effect on ischemic eye diseases such as Glaucoma and Diabetic Retinopathy is provided.

In another aspect of the invention, the method of administrating the vasodilators locally to the eye is provided. In particular topical eye drops, intravitreal delivery and gene therapy vectors are provided.

Therefore, the present invention relates to a novel way or mechanism of reversing the devastating effects of vasoconstriction in the retina, e.g. by reversing the cause of damage by upregulation of vasoconstrictive peptides such as endothelin-1 (ET-1). The Examples show *in vivo* data describing the proof of concept that single administration of the therapeutic CGRP peptide can reverse the ischemic damage caused by ET-1 induced vasoconstriction and thus the stop the initiation of vision deficit in the ischemic eye.

Thus, a presently preferred embodiment of the invention relates to treatment with Calcitonin gene-related peptide (CGRP) which is able to block the functional deficit created by endothelin-1 induced ischemia in the eye.

### DETAILED DESCRIPTION

The inventors of the present invention have discovered a novel way of reversing the devastating effects of vasoconstriction of retinal blood flow, which may be a common feature of several retinal diseases. Thus, the development of therapeutics or treatment schemes that has the potential of preventing the progressive neurodegeneration that eventually may lead to blindness in such retinal diseases is in high demand.

The data shown herein discloses that treatment with intravitreal or topical CGRP is able to block the functional deficit created by endothelin-1 induced ischemia in the eye. The application of this vasodilator is effective, long-lasting and without observable side effects.

The mechanism behind this prevention is probably caused by I) CGRP causing arterial smooth muscle relaxation, II) possible antagonistic effects of arterial ET-1 binding, and III) protective effect on retina possible through receptors in the nerve fiber layer.

In other tissues (lung, penis, heart) there are several publications that show CGRP protection of endothelin-1 induced ischemia that occurs with aging. The translation of these protective effects to become potential therapies have failed for other organs, because of inability to target this compound to the area of ischemia following systemic administration of this compound. For example, after systemic administration during a heart attack, healthy arteries can dilate and steal blood flow from the ischemic artery.

This is however in great contrast to the potential application in the eye. Since the eye is a closed compartment, one can apply the vasodilator locally. Thus, in one or more exemplary embodiments, the present invention relates only to the application of the vasodilator to the ischemic eye, leading to no systemic effects.

### Vasodilator

In the present context, a vasodilator is a compound that dilates blood vessels. They typically affect the smooth muscles in the walls of the arteries and the veins (directly or indirectly), preventing the muscles from tightening and the vessels from narrowing. The effect of a vasodilator is the widening of blood vessels resulting from relaxation of smooth muscle cells within the vessel walls, and increased blood flow. Inducing vasodilation and increasing the blood flow, will result in an increase in nutrients to the target tissue. Tissues in high demand of nutrients such as the retina, is at high risk of ischemia caused by vascular constriction.

Vasodilators work mainly either by lowering intracellular calcium concentration or by dephosphorylation (really substitution of ATP for ADP) of myosin. Dephosphorylation by myosin light-chain phosphatase and induction of calcium symporters and antiporters that pump calcium ions out of the intracellular compartment both contribute to smooth muscle cell relaxation and therefore vasodilation. This is accomplished through reuptake of ions into the sarcoplasmic reticulum via exchangers and expulsion across the plasma membrane.

There are four main intracellular stimuli that can result in the vasodilation of blood vessels: Hyperpolarization-mediated (e.g. Potassium channel opener), reducing intracellular calcium (e.g. Calcium channel blocker), cAMP-mediated, and cGMP-mediated (Nitrovasodilator). The specific mechanisms to accomplish these effects vary from vasodilator to vasodilator.

The vasodilators of the present invention may be an endogenous substance or an exogenous drug.

A vasodilator according to the present invention has an effect on retinal ischemic disorders by reversal of the constriction of the vasculature locally in the eye. Vasoconstriction locally in the retina in the present context is the narrowing of blood vessels resulting from contraction of the muscular wall of the vessels. In particular, opposite to classic systemic effects, the large arteries and small arterioles in the retina are typically targeted. Further, the application of a vasodilator, according to the present invention, to the choroid vasculature which supplies the posterior part of the retina will also increase the nutrient gradient locally in the retina.

The vasodilators of the invention include any medication being a compound or a composition that could mediate a local or non-systemic dilation of a retinal blood vessel when applied locally. The mechanism could for example be the activation of a receptor which induces vasodilation of the artery where the receptor is localized. For example, when using albino rats one can see through the eye and observe ciliary artery vasodilation in the back of the eye.

In one or more exemplary embodiments, the vasodilator dilates the ciliary artery with at least 20% as visualized with fundus imaging 10 minutes after application to an albino rat. The vasodilators with such high potency are considered able to reverse ischemia.

In one or more exemplary embodiments, the vasodilator reduces the retinal ischemic damage for both the photoreceptors (A-wave amplitude) and the Muller and ON-bipolar cells (B-wave amplitude) by at least 10%, when measured by Electroretinography in the mammal compared to the ET-1 induced ischemia alone, at day 3 and at day 21 after the vasodilator is first applied to said mammal.

Although, the eyes of different mammals vary in size, refractive properties, retinal vasculature and visual photopigments, the retinal cellular composition and thickness are conserved. Mammalian rod photoreceptors are similar: all contain rhodopsin, but size varies by animal size and diurnal/nocturnal behaviors. Related to the vascular organization, both human and rodent inner retinal blood supply is from central and cilioretinal arteries and the outer retina is supplied by the choriocapillaris, unlike the rabbit retina. A great animal for testing vasodilators is the rat which is widely used in experimental work, as the rat provides a rodent model for human ocular diseases and for toxicologic screening.

In one or more exemplary embodiments, the vasodilator reduces the retinal ischemic damage for both the photoreceptors (A-wave amplitude) and the Muller and ON-bipolar cells (B-wave amplitude) by at least 10%, when measured by Electroretinography in a Sprague Dawley rat, compared to the ET-1 induced ischemia alone at day 3 and at day 21 after an Endothelin-1 induced ischemic event.

### The vasodilator target

The vasodilators of the present invention improve blood flow locally to the eye. The retina has dual blood flow, consisting of retinal and choroid vasculature, both of which are supplied by the ocular vasculature. The vasodilator can increase the nutrient availability in the retina by targeting both systems.

The vasodilator will increase the flow to the whole eye, when applied outside the retina, including but not limited to topical or subtenon application. Said application targets ocular vasculature including, but limited to the ciliary artery, and the choroid vasculature which branches of the ciliary artery. This increases blood flow to the posterior retina and provide further nutrients. This can compensate for lack of flow in retinal vasculature.

In one or more exemplary embodiments, the vasodilator targets the ocular vasculature.

The vasodilator will increase retinal arterial flow directly in the inside of the eye, when applied intravitreally. This will increase the flow to the inner retina relieving the constriction and the ischemia.

In one or more exemplary embodiments, the vasodilator targets the retinal vasculature.

The vasodilators of the present invention ensure dilation activating receptors on cells regulating blood flow. In one or more exemplary embodiments, the vasodilator targets the smooth muscle cells (SMC), pericytes and/or the endothelia cells.

In a presently preferred embodiment, the vasodilator targets the smooth muscle cells. The smooth muscle cell is the direct cause of vasoconstriction of larger arteries and arterioles. The smooth muscle cell contracts to limit arterial diameter. The vasodilator in the present invention, targets receptors on the smooth muscle cell that reverse the constriction to increase arterial diameter.

In a presently preferred embodiment, the vasodilator targets the pericytes. Pericytes can regulate blood flow at the capillary level. For the retina, pericytes constrict capillaries when their membrane potential is altered to cause calcium influx. Applying said vasodilator to pericytes, will prevent the constriction and dilate capillaries before upstream arteriole dilation occurs.

In a presently preferred embodiment, the vasodilator targets the endothelia cells. Endothelial cells regulate smooth muscle and pericyte constriction by the release or transfer of local factors following their activation. Applying said vasodilator to the endothelial cells, will lead to indirect relaxation of the smooth muscle or pericyte and increase the arterial, arteriole or capillary diameter.

### Ciliary arteries

The ciliary arteries are divisible into three groups, the long posterior, short posterior, and the anterior. The short posterior ciliary arteries, from six to twelve in number, arise from the ophthalmic. The long posterior ciliary arteries, two for each eye, pierce the posterior part of the sclera at some little distance from the optic nerve. The anterior ciliary arteries are derived from the muscular branches of the ophthalmic artery.

In one or more exemplary embodiments, the vasodilator targets the ciliary artery and its branching choroid vasculature (external version).

### Retinal vasculature

The inner retina is supplied from the retinal vasculature, which gets its input from the central retinal artery. The venous part of the retinal circulation is arranged in a similar way. The central retinal vein leaves the eye through the optic disc and drains blood into the cavernous sinus. The retinal vasculature comprises the central retinal artery, retinal arteries, retinal arterioles, retinal capillaries, retinal venous arterioles, retinal veins, and the central retinal vein.

In one or more exemplary embodiments, the vasodilator targets the retinal vasculature (internal version).

### Vasculature posterior to the eye

In the present context, the vasculature posterior to the eye includes the ophthalamic artery, the long posterior ciliary artery, the short posterior ciliary artery, and the anterior ciliary artery, their associated veins, including but not limited to the scleral veins, the vorticois vein, the inferior opthalamic vein, superior opthalamic vein and their branching choroid vasculature such as, but not limited to, choroid arteries, arterioles, capillaries and veins.

In one or more exemplary embodiments, the vasodilator targets the vasculature posterior part of the eye.

### Specific vasodilators

Not all potential vasodilators provide a therapeutic effect locally in the eye. The inventors for example show in data set that sodium nitroprusside, although dilating the ciliary artery is not capable of mediating a protective effect for ischemia in the eye.

In one or more exemplary embodiments, the vasodilator for use in reversing the effects of vasoconstriction in the retina in a mammal, is a vasodilator that acts via the calcitonin receptor-like receptor (CRLR/RAMP-1) and wherein the vasodilator has a peptide motif in the N-terminal end having at least 99% identity to SEQ ID NO: 9 and a threonine (T) in amino acid position 6, and has at least 75% amino acid sequence similarity with SEQ ID NO: 2.

As shown in Example 1, adding CGRP intravitreally or topically caused a dilation of the ciliary artery. Intravitreal CGRP dilated the ciliary artery to 61 ± 12 % increase from baseline, before the injection of ET-1. Topical CGRP also increased the diameter (102 ± 21 % increase from baseline), visualized after the injection of intravitreal ET-1. After applying ET-1, the ciliary artery of the eye pretreated with intravitreal CGRP had a diameter of 9 ±15 % above baseline, compared to ET-1 plus vehicle where there was a contraction (-29 ±9% from baseline).

These observations indicate that vasodilators, in particular CGRP, may be use in the treatment of a retinal ischemic disorder in a mammal.

As shown in Example 1, at day 21, photoreceptor activity, represented by the A-wave amplitude, showed a significant functional deficit when comparing ET-1 induced ischemic right eyes (5µL 500µM, n = 9) and the control left eyes.

In relation to retinal function, adding CGRP intravitreally (5µL 200µM, n=11) or topically (10µL, 200 µM, n=8) had a strong protective effect. Applying CGRP reduced the ET-1 induced damage greatly at day 21, with the average A-wave amplitude when applied intravitreally being 545 ± 45 µV or applied topically being 511 ± 100 µV compared to the ET-1 control eye (457 ± 40 µV). Similarly, CGRP reduced the ET-1 induced damage on the bipolar cells at day 21, with the B-wave amplitude when applied intravitally being 1181 ± 202 µV) or applied topically being 1088 ± 411 µV compared to the ET-1 ischemic right eye (804 ± 188 µV).

This shows that in particular CGRP has a strong therapeutic effect on retinal ischemic disorder in a mammal, and thus in one or more presently preferred exemplary embodiments, the vasodilator is Calcitonin gene-related peptide (CGRP) or targets the calcitonin receptor-like receptor complex (CRLR/RAMP1). In the present context, then is meant by an CRLR/RAMP 1 agonist is a compound which (1) has a high affinity e.g., a K_{D} of less than 1 µM, such as but not limited to less than 0,9 µM, less than 0,8 µM, less than 0,7 µM, less than 0,6 µM, less than 0,5 µM, less than 0,4 µM, less than 0,3 µM, less than 0,2 µM, or less than 0,1 µM and (2) causes cAMP accumulation in a cell line that expresses the CRLR/RAMP1 receptor with an EC₅₀ of less than 10 µM, such as but not limited to less than 9 µM, less than 8 µM, less than 7 µM, less than 6 µM, less than 5 µM, less than 4 µM, less than 3 µM, less than 2 µM, less than 1 µM, less than 0,9 µM, less than 0,8 µM, less than 0,7 µM, less than 0,6 µM, less than 0,5 µM, less than 0,4 µM, less than 0,3 µM, less than 0,2 µM, or less than 0,1 µM.

Binding affinity is typically measured and reported by the equilibrium dissociation constant (K_{D}), which is used to evaluate and rank order strengths of bimolecular interactions. The smaller the K_{D} value, the greater the binding affinity of the ligand for its target. K_{D} may be determined by several methods known within the art for example ELISAs, gel-shift assays, pull-down assays, equilibrium dialysis, analytical ultra-centrifugation, SPR, spectroscopic assays and isothermal titration calorimetry. One example is binding to crude synaptosomal membranes for example from rat brains. Binding assays are conducted in siliconized glass tubes in a final assay volume of 250 µL containing 25-100 µg P2 membrane protein and 10-40 pmol/L radiolabel. (1251)-aCGRP (e.g. 2,200 Ci/mmol) is used as the radiolabel and nonspecific binding defined as remaining binding in the presence of either 10 µmol/L MK3207 or 1 µmol/L BIBN4096BS.

EC₅₀ for cAMP accumulation could for example be tested by Chinese hamster ovary (CHO) cells were stably transfected with calcitonin receptor-like receptor (CRLR), receptor activity modifying protein-1 (RAMP1) and CRE luciferase (cAMP responsive element for control of luciferase gene expression). Luciferase is a reporter-gene reporting accumulation of intracellular cAMP). Agonists are diluted in assay media before added to cells for stimulation. EC₅₀ can be determined by concentration-response curves fitted by use of GraphPad Prism software.

### Receptor target

Calcitonin gene-related peptide (CGRP), amylin and adrenomedullin (AM) belong to the same family of peptides and target the same receptor, the calcitonin receptor-like receptor (CRLR), with receptor specificity being determined by receptor-activity-modifying proteins (RAMPs). Three different RAMPs have been described in human tissue, RAMP1, RAMP2 and RAMP3. Co-expression of RAMP1 and CRLR forms a CGRP-receptor, whereas co-expression of RAMP2 or RAMP3 and CRLR form an AM receptor.

In one or more exemplary embodiments, the vasodilator targets the CGRP activated vasodilation system. CGRP or derivatives therefore activate receptors that consist of calcitonin receptor-like receptor (CRLR) linked to an essential receptor activity modifying protein (RAMP) that is necessary for full functionality. CGRP is a highly potent vasodilator, binding to said receptor on the smooth muscle cells, endothelium or pericytes, increasing the cAMP and thereby leading to direct or indirect increase in arterial diameter.

In one or more exemplary embodiments, the vasodilator targets the CGRP activated vasodilation system on the CRLR/RAMP 1 receptor. Examples of vasodilators targeting the targets the CGRP activated vasodilation system on the CRLR/RAMP 1 receptor includes Calcitonin gene-related peptide (CGRP), amylin, and adrenomedullin or derivatives/analogues thereof.

In the present context, an analogue of the peptides described herein is a compound having a structure similar to that of the natural peptide but differing from it in respect to a certain component.

In the present context, a derivative is a compound that is derived from a similar compound by a chemical reaction.

### Sequence identity

Proteins acting via the CRLR/RAMP 1 receptor have a strong degree of amino acid sequence similarity. Thus, in one or more exemplary embodiments, the vasodilators can be a peptide sharing at least 25 % amino acid sequence similarity with SEQ IN NO 1, such as being at least 30%, 35%, 40%, 45%, 50%, 55%, 60%, 65%, 70%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or being at least 99% identical with SEQ IN NO 1.

Thus, in one or more exemplary embodiments, the vasodilators can be a peptide sharing at least 75 % amino acid sequence similarity with SEQ IN NO 2, such as being at least 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or being at least 99% identical with SEQ IN NO 2.

Thus, in one or more exemplary embodiments, the vasodilators can be a peptide sharing at least 25 % amino acid sequence similarity with SEQ IN NO 3, such as being at least 30%, 35%, 40%, 45%, 50%, 55%, 60%, 65%, 70%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or being at least 99% identical with SEQ IN NO 3.

Thus, in one or more exemplary embodiments, the vasodilators can be a peptide sharing at least 25 % amino acid sequence similarity with SEQ IN NO 4, such as being at least 30%, 35%, 40%, 45%, 50%, 55%, 60%, 65%, 70%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or being at least 99% identical with SEQ IN NO 4.

Thus, in one or more exemplary embodiments, the vasodilators can be a peptide sharing at least 25 % amino acid sequence similarity with SEQ IN NO 5, such as being at least 30%, 35%, 40%, 45%, 50%, 55%, 60%, 65%, 70%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, being at least or 99% identical with SEQ IN NO 5.

Thus, in one or more exemplary embodiments, the vasodilators can be a peptide sharing at least 25 % amino acid sequence similarity with SEQ IN NO 6, such as being at least 30%, 35%, 40%, 45%, 50%, 55%, 60%, 65%, 70%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, being at least or 99% identical with SEQ IN NO 6.

Thus, in one or more exemplary embodiments, the vasodilators can be a peptide sharing at least 25 % amino acid sequence similarity with SEQ IN NO 7, such as being at least 30%, 35%, 40%, 45%, 50%, 55%, 60%, 65%, 70%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or being at least 99% identical with SEQ IN NO 7.

Thus, in one or more exemplary embodiments, the vasodilators can be a peptide sharing at least 25 % amino acid sequence similarity with SEQ IN NO 8, such as being at least 30%, 35%, 40%, 45%, 50%, 55%, 60%, 65%, 70%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or being at least 99% identical with SEQ IN NO 8.

### Peptide

A CGRP activated vasodilation acts via the calcitonin receptor-like receptor. Thus, the vasodilator targets the CGRP activated vasodilation, wherein the CGRP activated vasodilation is mediated by receptors activated by CGRP including but not limited to CRLR/RAMP1. Cell surface receptors act in cell signaling by receiving (binding to) extracellular molecules, here a peptide or a small protein, where the basic distinguishing factors are size and structure. Traditionally, peptides are defined as molecules that consist of between 2 and 50 amino acids, whereas proteins are made up of 50 or more amino acids.

In one or more exemplary embodiments, the vasodilator is a peptide acting via the CRLR/RAMP 1 receptor. In one or more exemplary embodiments, the peptide is selected from the group consisting of peptides analogues originating from CGRP or derivatives thereof. Said peptide binds to the CRLR/RAMP 1 receptor on smooth muscle cells, leading to arterial dilation. Such peptides are easily identifiable to a person skilled in the art. In the present context, a compound that binds to the CRLR/RAMP 1 receptor on smooth muscle cells, leading to arterial dilation is a compound which (1) has a high affinity (e.g., a K_{D} of less than 1 µM) tested on a blood vessel (or blood vessel preparation ) that is shown to express the CRLR/RAMP1 receptor (e.g by PCR or immunohistochemistry) and (2) causes vasodilation of said blood vessel with an EC₅₀ of less than 10 µM.

One example is binding to blood vessel membranes. Binding assays are conducted in siliconized glass tubes in a final assay volume of 250 µL containing 25-100 µg P2 membrane protein and 10-40 pmol/L radiolabel. (1251)-aCGRP (e.g. 2,200 Ci/mmol) is used as the radiolabel and nonspecific binding defined as remaining binding in the presence of either 10 µmol/L MK3207 or 1 µmol/L BIBN4096BS.

EC₅₀ for vasodilation could for example be tested in a rat brain artery that express calcitonin receptor-like receptor (CRLR), receptor activity modifying protein-1 (RAMP1). Arteries are mounted in an arterial myograph and preconstricted (for example by a solution containing 30 mM K⁺ or a constrictive agonist). The compound is added in a cumulative manner and vasodilation is observed as the arterial force is reduced. EC₅₀ can be determined by concentration-response curves fitted by use of GraphPad Prism software.

### Calcitonin gene-related peptide (CGRP)

Calcitonin gene-related peptide (CGRP) is a member of the calcitonin family of peptides. Calcitonin gene-related peptide (CGRP) is a 37 amino acid neuropeptide belonging to the calcitonin (CT) family of peptides. This family also includes amylin, the calcitonin receptor-stimulating peptides, and the adrenomedullins (AMs).

CGRP is a highly potent vasodilator, a microvascular vasodilator, which has a potency that is -10-fold higher than the most potent prostaglandins and 10-100 times greater than other vasodilators such as ACh. With other members of the CT family, it shares an N-terminal disulphide-bonded ring which is essential for biological activity, an area of potential a-helix, and a C-terminal amide.

CGRP binds to the calcitonin receptor-like receptor (CLR) in complex with receptor activity-modifying protein 1 (RAMP1), a member of the family B (or secretin-like) GPCRs. It can also activate other CLR or calcitonin-receptor/RAMP complexes.

In one or more exemplary embodiments, the vasodilator is a Calcitonin gene-related peptide (CGRP) or analogues thereof.

This 37 amino acid peptide comprises the N-terminal ring that is required for receptor activation (residues 1-7); an a-helix (residues 8-18), a region incorporating a b-bend (residues 19-26) and the C-terminal portion (residues 27-37), that is characterized by bends between residues 28-30 and 33-34. A few residues have been identified that seem to make major contributions to receptor binding and activation, with a larger number contributing either to minor interactions (which collectively may be significant), or to maintaining the conformation of the bound peptide. It is not clear if CGRP follows the pattern of other family B GPCRs in binding largely as an a-helix.

### The role of residues 1-7

Removal of residues 1-7 creates CGRP8-37, the prototypical CGRP antagonist. This binds approximately 10-fold less potently than CGRP and has lost all efficacy. Thus, residues 1-7 have a dual role in activating the receptor, contributing to the overall affinity of bound CGRP, and stabilizing the helix, as described in British Journal of Pharmacology (2013) 170 1308-1322.

Thus, in one or more exemplary embodiments, the vasodilator is a peptide comprising a motif in the N-terminal end having at least 50% identity to SEQ ID NO: 9 (XCXTATCXT), such as at least 51%. 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or having at least 99% identity to SEQ ID NO: 9 (XCXTATCXT).

Taken as a whole, some degree of modification is possible for most residues in 1-9, but it is likely that each contributes a modest amount to the binding or efficacy for CGRP.

In one or more exemplary embodiments, the motif has an Ala (A), Ser (S) or Gly (G) in position 1
In one or more exemplary embodiments, the motif has a Cystein (C) in position 2
In one or more exemplary embodiments, the motif has an Asn (N) or Asp (D) in position 3
In one or more exemplary embodiments, the motif has an Thr (T) in position 4.

In one or more exemplary embodiments, the motif has an Ala (A) or Ser (S) in position 5.

Nevertheless, Thr6 is probably a key residue for activation for all members of the CT family of peptides. Thus, in one or more exemplary embodiments, the motif has a Thr (T) in position 6.

In one or more exemplary embodiments, the motif has a Cystein (C) in position 7

In one or more exemplary embodiments, the motif has an Ala (A) or Val (V) in position 8.

In one or more exemplary embodiments, the motif has a Thr (T) in position 9.

### The disulphide-bonded loop

CGRP has several key structural features, most prominently a disulphide bond between residues 2 and 7 and a C-terminal amide.

Thus, in one or more exemplary embodiments, the vasodilator is a peptide comprising a motif in the N-terminal end having at least 50% identity to SEQ ID NO: 9 (XCXTATCXT), such as at least 51%. 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or having at least 99% identity to SEQ ID NO: 9, and wherein Cys2 and Cys7 are cyclised with a disulfide bond.

In one or more exemplary embodiments, the vasodilator is a peptide comprising a motif in the N-terminal end having at least 50% identity to SEQ ID NO: 9, such as at least 51%. 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or having at least 99% identity to SEQ ID NO: 9 with the proviso that the 1 and 7 cystine residues are not replaced with alpha-amino suberic acid (Asu).

In one or more exemplary embodiments, the vasodilator is a peptide comprising a motif in the N-terminal end having at least 50% identity to SEQ ID NO: 9, such as at least 51%. 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or having at least 99% identity to SEQ ID NO: 9, and wherein Cys2 and Cys7 are cyclised with a disulfide bond with the proviso that the peptide is not Elcatonin, a synthetic analogue of eel calcitonin.

In one or more exemplary embodiments, the vasodilator is a peptide comprising a motif in the N-terminal end having at least 50% identity to SEQ ID NO: 9, such as at least 51%. 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or having at least 99% identity to SEQ ID NO: 9, and wherein Cys2 and Cys7 are cyclised with a disulfide bond with the proviso that the peptide is not SEQ ID NO: 10.

In one or more exemplary embodiments, the vasodilator is a peptide comprising a motif in the N-terminal end having at least 50% identity to SEQ ID NO: 9, such as at least 51%. 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or having at least 99% identity to SEQ ID NO: 9, wherein Cys2 and Cys7 are cyclised with a disulfide bond, and wherein the with the proviso that the 1 and 7 cystine residues are not replaced with alpha-amino suberic acid (Asu).

Homologues, derivatives and fragments of the vasodilating peptides according to the present invention all have analogous biological activity. Homologues, derivatives and fragments, in the present context, comprise at least 50% identity to SEQ ID NO: 9, and wherein Cys2 and Cys7 are cyclised with a disulfide bond, which may be further modified by changing one or more amino acids in order to optimize the desired biological activity, without altering the essential vasodilating function of the peptide.

In one or more exemplary embodiments, the vasodilator is CGRP. In the present context CGRP include pre-proCGRP, derivatives of CGRP, fragments of CGRP, any molecule containing the CGRP peptide sequence or a molecule containing a modified CGRP peptide sequence. Typically, variations of CGRP would include one or more of conservative amino acid substitution(s), non-conservative amino acid substitution(S), and/or addition of linkers to make it more stable, see for example WO11051312.

### Small molecule

Pharmacology usually restricts the term "small molecule" to molecules that bind specific biological macromolecules and acts as an effector, altering the activity or function of the target. Small molecules can have a variety of biological functions or applications, serving as cell signaling molecules, drugs in medicine, pesticides in farming, and in many other roles.

In another or more embodiments, the vasodilator is a small molecule. Said small molecule binds to the CRLR/RAMP1 receptor, leading to activation of the receptor. Activation of the receptor leads to vasodilation and increase in arterial diameter.

### CGRP compounds

Native CGRP has a half-life of less than 30 minutes and a short duration of pharmacological actions after CGRP infusions is evident. Thus, the pharmacological usefulness of CGRP may require the generation of CGRP analogues with prolonged action and some effects may even only be obtained with prolonged analogues.

In one or more exemplary embodiments, the vasodilator is selected from the group consisting of compounds of the general formula Z-Y-X, wherein X designates a CGRP compound from which, formally, a hydrogen has been removed from one of the amino groups present in an amino acid residue; Y is a linker elected from the group consisting of the six groups of the following formulae: 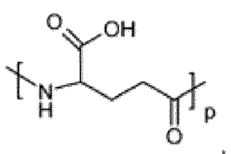 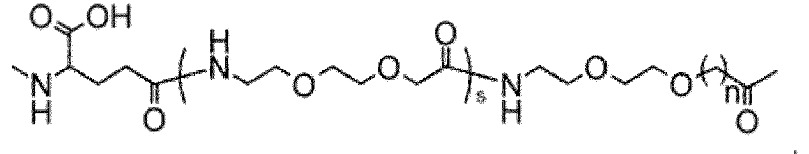 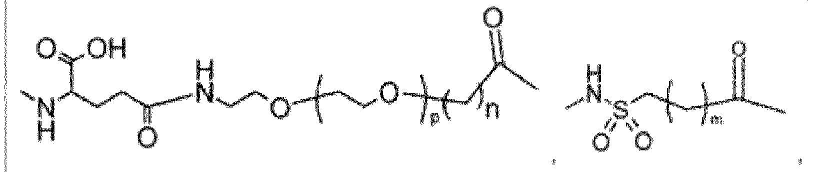 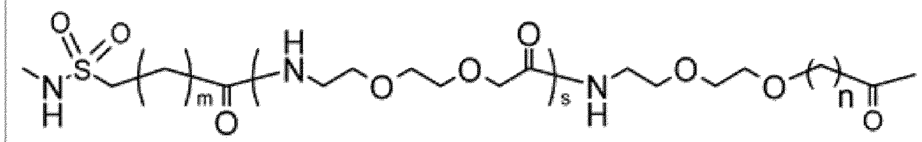 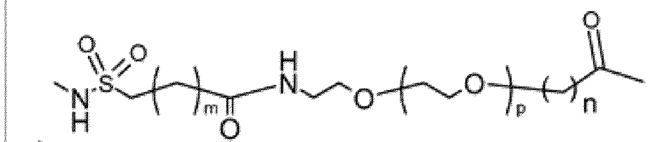 land ,
wherein m is 0, 1, 2, 3, 4, 5 or 6; n is 1, 2 or 3; s is 0, 1, 2 or 3; and p is 1, 2, 3, 4, 5, 6, 7, 8, 9, 10, 1 1, 12, 13, 14, 15, 16, 17,18, 19, 20, 21, 22 or 23, and wherein the carbonyl end of these Y moieties is connected to the CGRP compound (designated X) and the amino end of these Y moieties is connected to Z; and Z is HOOC-(CH₂)_{q}-(CO)ᵣ; wherein q is an integer in the range 10 to 20 and r is o or 1.

In one or more exemplary embodiments, q is an integer in the range 12 to 18, such as in the range 14 to 16.

In one or more exemplary embodiments, r is 1, and q is an integer in the range 10 to 20, such as in the range 12 to 18, e.g. in the range 14 to 16.

In one or more exemplary embodiments, the CGRP compound has the general formula I (SEQ ID NO: 11): X₁CX₂TX₃TCX₄TX₅RLAX₆X₇LX₈RSGGX₉X₁₀X₁₁X₁₂X₁₃FVPTX₁₄VX₁₅X₁₆X₁₇X₁₈F, wherein X₁ is Ala or Ser, X₂ is Asp or Asn, X₃ is Ala or Ser, X₄ is Val or Ala, X₅ is His or Gln, X₆ is Gly or Asp, X₇ is Leu or Phe, X₈ is Ser, Asn or Arg, X₉ is Val, Ile, Met or Leu, X₁₀ is Val, Ala, Leu or Gly, X₁₁ is Lys, Ser, Asn or His, X₁₂ is Ser, Asn, Asp, Pro, X₁₃ is Asp or Asn, X₁₄ is Asp or Asn, X₁₅ is Gly or Ser, X₁₆ is Ala or Ser, X₁₇ is Glu, Gin, Lys or Asn, X₁₈ is Ala or Ser, and the carboxy group in the C terminal amino acid residue is, optionally, amidated, and, in formula I, the specific amino acid residues are indicated by the usual one letter codes for the amino acids; and said CGRP compounds have the usual intramolecular disulphide bridge between the two Cys residues.

In one or more exemplary embodiments the CGRP compound has the general formula II (SEQ ID NO: 4): X₁CX₂TATCVTHRLAX₆LLX'₈RSGGX'₉X'₁₀KX'₁₂NFVPTX₁₄VGSX'₁₇AF, wherein X₁ is Ala or Ser, X₂ is Asp or Asn, X₆ is Asp or Gly, X'₈ is Arg or Ser, X'₉ is Val or Met, X'₁₀ is Val or Leu, X'₁₂ is Asp, Asn or Ser, X₁₄ is Asp or Asn, X'₁₇ is Glu or Lys, and the carboxy group in the C terminal amino acid residue is, optionally, amidated.Thus, in one or more embodiments, the vasodilator is pre-proCGRP, derivatives of CGRP, fragments of CGRP, any molecule containing the CGRP peptide sequence or a molecule containing a modified CGRP peptide sequence.

In humans CGRP exists in two forms, α-CGRP and β-CGRP. α-CGRP is a 37-amino acid peptide and is formed from the alternative splicing of the calcitonin/CGRP gene located on chromosome 11. The less-studied β-CGRP differs in three amino acids (in humans) and is encoded in a separate gene in the same vicinity.

In one or more embodiments, the vasodilator is α-CGRP or fragments thereof.

### Treatment

A method for treating a retinal ischemic disorder in a subject comprising administering a vasodilator as described herein as a main component locally to the retina.

"Treating" or "treatment" as used herein refers to reducing or eliminating the cause of a disease in a subject suffering from the disease, delaying or stopping the progression of the disease, and/or alleviating, alleviating, ameliorating or eliminating the symptoms of the disease, such as abnormalities in the visual function.

The vertebrate retina has ten distinct layers:
Inner limiting membrane - basement membrane elaborated by Muller cells
Nerve fiber layer - axons of the ganglion cell nuclei (note that a thin layer of Muller cell footplates exists between this layer and the inner limiting membrane)
Ganglion cell layer - contains nuclei of ganglion cells, the axons of which become the optic nerve fibers for messages and some displaced amacrine cells
Inner plexiform layer - contains the synapse between the bipolar cell axons and the dendrites of the ganglion and amacrine cells
Inner nuclear layer - contains the nuclei and surrounding cell bodies (perikarya) of the amacrine cells, bipolar cells, and horizontal cells
Outer plexiform layer - projections of rods and cones ending in the rod spherule and cone pedicle, respectively. These make synapses with dendrites of bipolar cells
In the macular region, this is known as the Fiber layer of Henle
Outer nuclear layer - cell bodies of rods and cones
External limiting membrane - layer that separates the inner segment portions of the photoreceptors from their cell nucleus
Layer of rods and cones - layer of rod cells and cone cells
Retinal pigment epithelium - single layer of cuboidal cells

Thus, in one or more exemplary embodiments, the method for preventing or treating a retinal ischemic disorder in a subject comprising administering a vasodilator or a viral vector capable of producing a vasodilator as described herein as a main component locally to at least one of the ten distinct layers of the retina.

Thus, in one or more exemplary embodiments, the method for preventing or treating a retinal ischemic disorder in a subject comprising administering a vasodilator as described herein as a main component locally to a tissue directly surrounding the retina.

In one or more exemplary embodiments, the administration is non-systemic.

The present invention relates to the treatment of retinal ischemic disorders in the sense of a cure of the disorder, as well as prevention of initiation of the retinal ischemic disorder, prevention of the progression of the retinal ischemic disorder.

The vast majority of diseases that cause catastrophic loss of vision do so as a result of ocular neovascularization. During normal retinal vascular development, vascular endothelial cells proliferate and migrate through the extracellular matrix in response to a variety of cytokines, leading to the formation of new blood vessels in a highly ordered fashion. During abnormal neovascularization of the iris, retina, or choroid, angiogenesis is unregulated and usually results in the formation of dysfunctional blood vessels. When these newly formed vessels leak fluid, hemorrhage, or are associated with fibrous proliferation, retinal edema, retinal/vitreous hemorrhage, or traction, retinal detachments may occur resulting in potentially catastrophic loss of vision. Neovascularization is a compensatory mechanism for reduced blood flow in the retina, which can be caused by ischemia. Thus, in one or more embodiments, the vasodilators of the present invention are useful in the treatment of neovascular eye diseases preventing the root cause.

### Retinal Ischemic Disorders

A retinal ischemic disorder according to the present invention relates to any disturbance of ocular blood flow having a medical effect in the retinal, and thus a method of treatment of a retinal ischemic disorder according to the present invention include increasing retinal tolerance time, reducing cell death during an ischemic event in the retina, reducing cell death following an ischemic event in the retina, treating an ischemic event in the retina, preventing an ischemic event in the retina, or a combination thereof.

In one or more exemplary embodiments, the treatment regime of the present invention relates to a method comprising administering a dose of a pharmaceutical composition comprising an effective amount of any of the vasodilators of the invention to a subject in need thereof. Thereby performing a treatment to resolve vascular constriction causing the ischemic event.

In one or more exemplary embodiments, the retinal ischemic disorder is selected from the group consisting of macular degeneration, diabetic eye disease, diabetic retinopathy, age-related macular degeneration, branch retinal vein occlusion, central retinal vein occlusion, central retinal artery occlusion, central serous retinopathy, diabetic retinopathy, giant cell arteritis, glaucoma, normotensive glaucoma, hypertensive retinopathy, thyroid eye disease, ischemic optic neuropathy, juvenile macular degeneration, macular edema, macular telangioctasia, retinal detachment, ocular hypertension, neovascularization, retinal vein occlusion, retinitis pigmentosa, retinopathy of prematurity, stargardt disease, retinal artery occlusion, and usher syndrome, or any combination thereof.

In one or more exemplary embodiments, the retinal ischemic disorder is selected from the group consisting of diabetic retinopathy, retinitis pigmentosa, glaucoma, normotensive glaucoma, ocular hypertension, neovascularization, retinal vein occlusion, and retinal artery occlusion.

In one or more exemplary embodiments, the retinal ischemic disorder is selected from the group consisting of diabetic retinopathy, glaucoma, normotensive glaucoma, and retinal artery occlusion.

In one or more exemplary embodiments, the retinal ischemic disorder is selected from the group consisting of glaucoma, normotensive glaucoma, and retinal artery occlusion.

In one or more exemplary embodiments, the retinal ischemic disorder is selected from the group consisting of diabetic retinopathy and retinal artery occlusion.

In one or more exemplary embodiments, the retinal ischemic disorder is selected from the group consisting of diabetic retinopathy, glaucoma, and normotensive glaucoma.

In one or more exemplary embodiments, the retinal ischemic disorder is selected from the group consisting of glaucoma and normotensive glaucoma.

In one or more exemplary embodiments, the retinal ischemic disorder is caused by Endothelin-1.

### Diabetic Retinopathy

Diabetic retinopathy is caused by damage to the blood vessels of the light-sensitive tissue at the back of the eye.

Diabetic retinopathy is the result of damage to the small blood vessels and neurons of the retina. The earliest changes leading to diabetic retinopathy include narrowing of the retinal arteries associated with reduced retinal blood flow; dysfunction of the neurons of the inner retina, followed in later stages by changes in the function of the outer retina, associated with subtle changes in visual function; dysfunction of the blood-retinal barrier, which protects the retina from many substances in the blood (including toxins and immune cells), leading to the leaking of blood constituents into the retinal neuropile.

Later, the basement membrane of the retinal blood vessels thickens, capillaries degenerate and lose cells, particularly pericytes and vascular smooth muscle cells. This leads to loss of blood flow and progressive ischemia, and microscopic aneurysms which appear as balloon-like structures jutting out from the capillary walls, which recruit inflammatory cells; and advanced dysfunction and degeneration of the neurons and glial cells of the retina.

In one or more exemplary embodiments, the retinal ischemic disorder is diabetic retinopathy.

### Retinitis Pigmentosa

Retinitis pigmentosa (RP) is a genetic disorder of the eyes that causes loss of vision. A variety of indirect symptoms characterize retinitis pigmentosa along with the direct effects of the initial rod photoreceptor degeneration and later cone photoreceptor decline. Findings related to RP have often been characterized in the fundus of the eye as the "ophthalamic triad". This includes the development of (1) a mottled appearance of the retinal pigment epithelium (RPE) caused by bone spicule formation, (2) a waxy appearance of the optic nerve, and (3) the attenuation of blood vessels in the retina.

In one or more exemplary embodiments, the retinal ischemic disorder is retinitis pigmentosa.

### Glaucoma

Glaucoma is a group of related eye disorders that cause damage to the optic nerve or the retinal ganglion cells or retinal ganglion nerve fiber layer that carries information from the eye to the brain. The damage to the optic nerve ganglion cells or retinal ganglion nerve fiber layer may be related to poor blood flow to the optic nerve, retina, or choroid. The most common type is open-angle glaucoma with less common types including closed-angle glaucoma and normal-tension glaucoma.

In one or more exemplary embodiments, the retinal ischemic disorder is Glaucoma.

### Normal Tension Glaucoma

Glaucoma is a disease affecting the optic nerve and can result in permanent, irreversible vision loss. While most cases of glaucoma are associated with higher than average eye pressures, normal-tension glaucoma (and low-tension glaucoma) is a unique condition in which glaucomatous optic nerve damage or damage to the retinal ganglion cells or retinal ganglion nerve fiber layer (optic neuropathy) occurs despite an average or below average eye pressure.

In one or more exemplary embodiments, the retinal ischemic disorder is normal-tension Glaucoma and/or low-tension Glaucoma.

### Ocular Hypertension

Ocular hypertension is not the same as glaucoma. With ocular hypertension, the optic nerve looks normal and there are no signs of vision loss. However, people with ocular hypertension are considered "glaucoma suspects." That means they should see their ophthalmologist regularly to be checked for glaucoma. Experimentally induced ischemia of the endothelial cells surrounding the aqueous drainage sites led to a rise in intraocular pressure (IOP) in monkey and pigeon eyes. Clinical conditions associated with a rise in IOP in human eyes, e.g. peripheral retinal detachments, subluxated lenses, and occlusion of the internal carotid artery, can result in ischemia of the endothelial cells lining Schlemm's canal. These findings led to the hypothesis that ischemia of the endothelial cells of Schlemm's canal induces hypertension in human eyes.

In one or more exemplary embodiments, the retinal ischemic disorder is ocular hypertension.

### Retinal Neovascularization

Neovascularization is the natural formation of new blood vessels, usually in the form of functional microvascular networks, capable of perfusion by red blood cells, that form to serve as collateral circulation in response to local poor perfusion or ischemia.

Neovascularization within the eye contributes to visual loss in several ocular diseases, the most common of which are proliferative diabetic retinopathy, neovascular age-related macular degeneration, and retinopathy of prematurity.

In one or more exemplary embodiments, the retinal ischemic disorder leads to retinal neovascularization.

### Retinal Vein Occlusion

Retinal vein occlusion (RVO) is a common cause of vision loss in older individuals, and the second most common retinal vascular disease after diabetic retinopathy. There are two distinct types, classified according to the site of occlusion: in central RVO (CRVO), the occlusion is at or proximal to the lamina cribrosa of the optic nerve, where the central retinal vein exits the eye. CRVO is further divided into the categories of perfused (non-ischemic) and nonperfused (ischemic), each of which has implications for prognosis and treatment.

In one or more exemplary embodiments, the retinal ischemic disorder is retinal vein occlusion.

In one or more exemplary embodiments, the retinal ischemic disorder is nonperfused retinal vein occlusion.

### Retinal Artery Occlusion

Retinal artery occlusion is an ophthalmic emergency that requires immediate evaluation and transfer to a stroke center. It is an obstruction of retinal blood flow that may be due to an embolus causing occlusion or thrombus formation, vasculitis causing retinal vasculature inflammation, traumatic vessel wall damage, or spasm. The lack of oxygen delivery to the retina during the blockage often results in severe vision loss in the area of ischemic retina. Patients often have concurrent silent ischemic stroke.

In one or more exemplary embodiments, the retinal ischemic disorder is retinal artery occlusion.

### Anterior Ischemic Optic Neuropathy

Anterior ischemic optic neuropathy (AION) is a sudden loss of vision due to an interruption of blood flow to the front (anterior) of the optic nerve, also known as the optic nerve head. The optic nerve's task is to carry visual information from the eye to the brain, which assembles this information into images. Ischemia occurs at the head of the optic nerve in relation with structural crowding of the nerve fibers, impairing perfusion and leading to optic disc edema.

In one or more exemplary embodiments, the retinal ischemic disorder is anterior ischemic optic neuropathy.

### Composition

The vasodilator can be provided in a pharmaceutical composition. The pharmaceutical composition can comprise pharmaceutically acceptable diluent(s), excipient(s), or carrier(s). The pharmaceutical compositions can include other medicinal or pharmaceutical agents, carriers, adjuvants, such as preserving, stabilizing, wetting or emulsifying agents, solution promoters, salts for regulating the osmotic pressure, and/or buffers. All methods well known in the art for making compositions.

Pharmaceutical compositions comprising a vasodilator of the present invention can be formulated for delivery locally to the eye.

Generally, the compounds of the invention are administered in a therapeutically effective amount in a pharmaceutically acceptable dosage form. The actual compound, i.e. the amount of active ingredient, will depend on a number of factors such as the severity of the disease to be treated, the age and relative health of the subject, the ability of the compound used, the route and form of administration and other factors.

The compound of the present invention is a topical eye drop that can be administered once or more a day, preferably 3 or 4 times a day. If the compound is an intravitreal injection, it will be injected for example only once a week by clinicians. If the compound is a viral vector, one single injection might be sufficient. All these factors are within the skill of the attending clinician.

Thus, in one or more exemplary embodiments, the present invention relates to a composition comprising a vasodilator according to the invention.

In one or more exemplary presently preferred embodiments, the present invention relates to a composition comprising a CGRP system targeted vasodilator.

### Neuroprotection

Since retinal ischemia is not limited to the lack of flow but also implies that the retina of the eye is involved in the disease or disorder. The retina comprises neurons, and as such retinal disorders as such are disorders in which neuritogenesis and/or neuroprotection are desirable.

The application of a vasodilator according to the invention such as but not limited to intravitreal CGRP, topical application of CGRP or production of CGRP from a viral vector, is combined with the intravitreal injection of a neuroprotectant. This further enhances the retinal function as flow is restored following the CGRP application/production and neurons/retina is further protected by the neuroprotectant.

In one or more exemplary embodiments, the composition further comprises a neuroprotection ingredient.

In one or more exemplary embodiments, the neuroprotection ingredient is administered simultaneously, separately or sequentially from the vasodilator.

In one or more exemplary embodiments, the neuroprotection ingredient is glial cell-line derived neurotrophic factor (GDNF), insulin like growth factor-1 (IGF-1), platelet-derived growth factor CC (PDGF-CC), serotonin analogues, neuropeptide Y fragments, brimonidine or basic fibroblast growth factor (FGF).

### Nucleic acid construct

Disclosed herein are nucleic acid constructs and expression vectors for enhancing the production of recombinant vasodilator such as a peptide and/or a protein having the treatment effect on the retinal ischemic disorders. In one or more exemplary embodiments, the nucleic acid construct encodes a peptide consisting of 15 to 55 contiguous amino acids.

In one or more exemplary embodiments, the nucleic acid construct encodes a peptide consisting of 15 to 50 contiguous amino acids.

In one or more exemplary embodiments, the nucleic acid construct encodes a peptide consisting of 15 to 33 contiguous amino acids.

In one or more exemplary embodiments, the invention relates to a delivery vehicle comprising the nucleic acid construct according to the present invention.

The delivery vehicle may be selected from the group consisting of: RNA based vehicles, DNA based vehicles, lipid based vehicles, polymer based vehicles, colloidal gold particles, virally derived DNA or RNA vehicles, adenoviruses, recombinant adeno-associated viruses (rAAV), retroviruses, lentiviruses, adeno-associated viruses, herpesviruses, vaccinia viruses, foamy viruses, cytomegaloviruses, Semliki forest virus, poxviruses, RNA virus vector and DNA virus vector.

In one or more exemplary embodiments, the delivery vehicle is an adenovirus, recombinant adeno-associated viruses or an adeno-associated virus.

In one or more presently preferred embodiments, the peptide or nucleic acid construct is administered directly into the eye, such as by means of intravitreal or subretinal administration or injection.

### Ocular drug delivery system

The major challenge faced by today's pharmacologist and formulation scientist is ocular drug delivery. Topical eye drop is the most convenient and patient compliant route of drug administration, especially for the treatment of anterior segment diseases. Delivery of drugs to the targeted ocular tissues is restricted by various precorneal, dynamic and static ocular barriers. Also, therapeutic drug levels are not maintained for longer duration in target tissues.

The pharmaceutical composition comprising a vasodilator according to the invention is preferably administered locally to an eye of the subject via an ocular drug delivery system. Thus, the present invention relates to an ocular drug delivery system comprising a vasodilator or a composition comprising a vasodilator.

An ocular drug delivery system in the present context can be selected from the group consisting of topical eye drop formulations, nucleic acid constructs, viral and non-viral gene therapy vectors, emulsion based formulations, suspensions, ophthalmic ointments, nanotechnology based ocular drug delivery systems (nanomicelles, nanoparticles, nanosuspensions), liposomes, monomers, multimers, dendrimers, in-situ gelling systems, contact lens, implants, slow release polymers, microneedles, microspheres, and cells capable of secreting a vasodilator in the eye.

In one or more exemplary embodiments, the ocular drug delivery system is capable of delivering the vasodilator to the retina, so the effect of the vasodilator is locally in the eye and not a systemic effect.

### Route of administration

The choice of formulation and route of administration of the ocular drug delivery system is based on a variety of factors such as drug dosage form and bioavailability of the drug substance. The medicament may be administered as a pharmaceutical composition by any one or a combination of two or more of the following routes: oral administration, transdermal administration, intranasal administration or by suppository administration, topical administration (subconjunctival, Tenon's capsule or transdermally), by instillation, by injection (intravitreal, subconjunctival, Tenon's capsule or intraperitoneally).

In one or more exemplary embodiments, the ocular drug delivery system is administrated by topical administration, intravitreal injection, intracameral administration, subconjunctival administration, subtenon administration, retrobulbar administration, posterior juxtascleral administration, or a combination thereof.

The choice of an ocular drug delivery system is targeted to the eye locally depends on many factors including the ocular bioavailability, particularly in the light of the blood retina barrier, where compounds cannot cross to the other side of the retinal vessel. Applying said medicament to the eye locally will improve local circulation. The medicament to be administered has a pharmaceutical composition by any one or a combination preferably the two following routes: topical administration or intravitreal injection.

In one or more exemplary embodiments, the ocular drug delivery system is administrated by topical administration or intravitreal injection.

### Liquid Dosage Form

Liquid dosage forms for administration may include pharmaceutically acceptable emulsions, solutions, suspensions, syrups and elixirs containing inert diluents commonly used in the art such as water. Such a composition may also contain adjuvants such as wetting agents, emulsifying and suspending agents, cyclodextrins and fragrances.

### Emulsions

An emulsion-based formulation approach offers an advantage to improve both solubility and bioavailability of drugs. There are two types of emulsions which are commercially exploited as vehicles for active pharmaceuticals: oil in water (o/w) and water in oil (w/o) emulsion systems. For ophthalmic drug delivery, o/w emulsion is common and widely preferred over w/o system. The reasons include less irritation and better ocular tolerance of o/w emulsion. Several studies have demonstrated applicability of emulsions in improving precorneal residence time, drug corneal permeation, providing sustain drug release and thereby enhancing ocular bioavailability.

### Suspensions

Suspension particles retain in precorneal pocket and thereby improve drug contact time and duration of action relative to drug solution. Duration of drug action for suspension is particle size dependent. Smaller particle size replenishes the drug absorbed into ocular tissues from precorneal pocket. While on the other hand, larger particle size helps retain particles for longer time and slow drug dissolution. Thus, an optimal particle size is expected to result in optimum drug activity.

### Ophthalmic Ointments

Ophthalmic ointments are another class of carrier systems developed for topical application. Ocular ointment comprises of mixture of semisolid and a solid hydrocarbon (paraffin) that has a melting point at physiological ocular temperature (34 °C). The choice of hydrocarbon is dependent on biocompatibility. Ointments help to improve ocular bioavailability and sustain the drug release.

### Liposome

The compounds of the present invention can also be administered in the form of liposomes. Liposomes are generally derived from phospholipids or other lipid substances, as is known in the art. Liposomes are formed by mono- or multilamellar hydrated liquid crystals dispersed in an aqueous medium. Any non-toxic, physiologically acceptable, metabolizable lipid capable of forming liposomes can be used. The composition in liposome form may contain stabilizers, preservatives, excipients and the like. Examples of lipids are natural and synthetic phospholipids and phosphatidylcholine (lecithin). Methods of forming liposomes are known in the art.

For ophthalmic applications, liposomes represent ideal delivery systems due to excellent biocompatibility, cell membrane like structure and ability to encapsulate both hydrophilic and hydrophobic drugs. Liposomes have demonstrated good effectiveness for both anterior and posterior segment ocular delivery.

### Compressed Gas

Compressed gases can be used to disperse the compounds of the invention in an aerosolized form. Suitable inert gases for this purpose are nitrogen, carbon dioxide and the like.

To overcome the ocular drug delivery barriers and improve ocular bioavailability, various conventional and novel drug delivery systems have been developed such as emulsion, ointments, suspensions, aqueous gels, nanomicelles, nanoparticles, liposomes, dendrimers, implants, contact lenses, nanosuspensions, microneedles, and *in situ* thermosensitive gels for the earlier mention ocular diseases. The inventors of the present invention have shown in the Examples that both traditional topical eye drops and more sophisticated appliance, for example through a microneedle to specific areas, provide the necessary therapeutic results - see e.g. the intravitreal delivery data.

### Topical Eye Drop

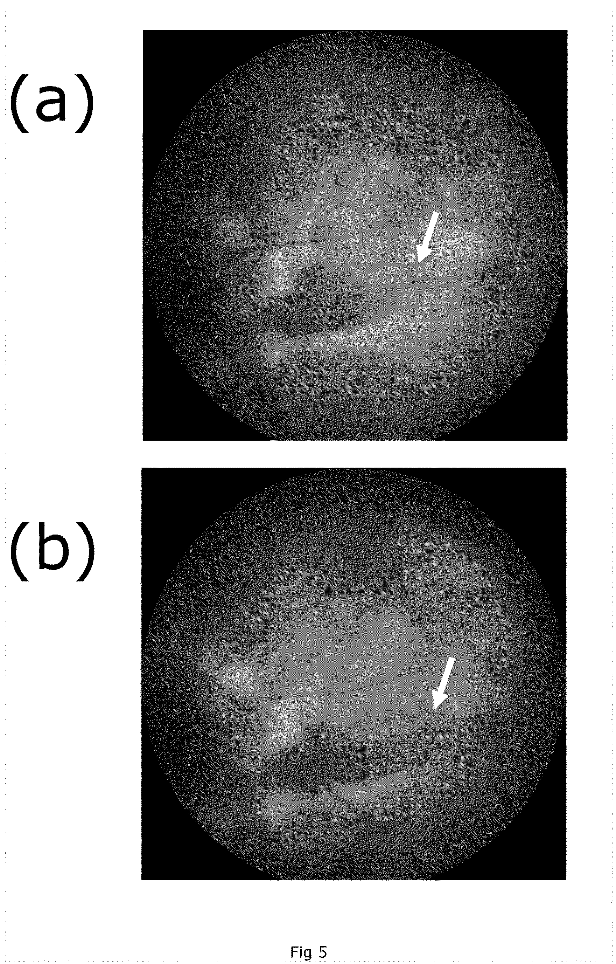

FIG. 5 shows the dilation of the ciliary artery when CGRP is applied topically to the eye of a Sprague dawley rat, where the artery dilated more than 20%. In Example 1, topical CGRP also increased the diameter (102 ± 21 % increase from baseline), visualized after the injection of intravitreal ET-1.

In one or more presently preferred embodiments, the ocular drug delivery system is a topical eye drop delivery system comprising a vasodilator as disclosed herein, preferably CGRP.

In certain embodiments, topical administration is by eye drops, subconjunctival injections, Tenon's capsule injections, or intravitreal injections.

The compounds of the present invention can also be administered in the form of eye drops. The ophthalmic preparation may contain an isotonizing agent such as sodium chloride or concentrated glycerin; a buffering agent such as sodium phosphate or sodium acetate; a surface active agent such as polyoxyethylene sorbitan monooleate, polyoxyl stearate 40, polyoxyethylene hardened castor oil; an agent; a stabilizer such as sodium citrate, sodium edetate and the like; a preservative such as benzalkonium chloride, paraben and the like, if necessary.

The pH may be within the allowable range for ophthalmic preparations, but it is usually within the range of 4 to 8. The ophthalmic ointment can be prepared using a commonly used base such as white petrolatum and liquid paraffin.

The appliance of the vasodilators locally is important, if systemic effects of the vasodilators is to be avoided. Thus, applying the ocular delivery system direct at or close to the observed ischemia is preferable. This can for example be achieved by applying the ocular delivery system comprising the desired vasodilator using topical ocular, retro bulbar, sub-tenon, sub-conjunctively, or intravitreally delivery.

### Microneedles

Microneedle-based technique is an emerging and minimally invasive mode of drug delivery to posterior ocular tissues. This technique may provide efficient treatment strategy for vision threatening posterior ocular diseases such as age-related macular degeneration, diabetic retinopathy and posterior uveitis.

This new microneedle-based administration strategy may reduce the risk and complications associated with intravitreal injections such as retinal detachment, hemorrhage, cataract, endophthalmitis and pseudoendophthalmitis. Moreover, this strategy may help to circumvent blood retinal barrier and deliver therapeutic drug levels to retina/choroid. Microneedles are custom designed to penetrate only hundreds of microns into sclera, so that damage to deeper ocular tissues may be avoided. These needles help to deposit drug or carrier system into sclera or into the narrow space present between sclera and choroid called "suprachoroidal space" (SCS). Puncturing of sclera and depositing drug solution or carrier systems in sclera or SCS may facilitate diffusion of drug into deeper ocular tissues, choroid and neural retina. For intraocular delivery of drugs, the application of microneedles surface coated with drugs has shown that surface coated drug was rapidly dissolved in scleral tissue indicating high scleral sulforhodamine deposition within microneedle hole. Nanoparticles suspensions and microparticles were also delivered into sclera by microneedles however; microparticles were delivered only in the presence of collagenase spreading enzymes and hyaluronidase. Studies demonstrated that hollow microneedles may be employed for scleral infusion of drug or micro/nanoparticles with minimal invasive route.

### Injectable Preparation

Injectable preparations, for example, sterile injectable aqueous or oleaginous suspensions, can be formulated according to the known art using suitable dispersing or wetting agents or suspending agents. The sterile injectable preparation may also be a sterile injectable solution or suspension in a non-toxic parenterally-acceptable diluent or solvent, for example as a solution in 1,3-propanediol. Acceptable vehicles and solvents that can be used are water, Ringer's solution and isotonic sodium chloride solution. In addition, sterile fixed oils are conveniently used as a solvent or dispersion medium. For this purpose, any grade fixed oil can be used including synthetic mono- or diglycerides. In addition, fatty acids such as oleic acid can be used in injectable formulations.

In one or more exemplary embodiments, the ocular drug delivery system is an injectable preparation comprising a vasodilator as disclosed herein, preferably CGRP.

In the Examples, intravitreal CGRP dilated the ciliary artery to 61 ± 12 % increase from baseline, before the injection of ET-1. After applying ET-1 the ciliary artery of the eye pretreated with intravitreal CGRP maintained a diameter of 9 ±15 % above baseline, compared to ET-1 plus vehicle where there was a contraction and reduced diameter (-29 ±9% from baseline).

### Intravitreal Delivery

FIG. 1 and FIG. 2 show some of the areas of injection where the invention has been applied locally in the eye. FIG. 8 shows the dilation of the ciliary artery induced by CGRP when applied intravitreally. FollowingET-1 (5µL, 500µM) injection the artery constricted. (a) The addition of CGRP (+dilator) maintained the diameter of the ciliary artery, even in the presence of 500 µM ET-1 (n=9-11). (b) The addition of CGRP, reduced the damage in the retina represented as function loss of photoreceptors cells (A-wave amplitudes) compared to the un-injected eye (n= 9-11). (c) Damage of the retina represented as function loss of bipolar cells (B-wave amplitudes) compared to the un-injected eye was also greatly improved when pretreated with CGRP, compared to ET-1 alone.

In one or more exemplary embodiments, the ocular drug delivery system is an intravitreal delivery.

Injections for intravitreal administration include isotonizing agents such as sodium chloride, buffering agents such as sodium phosphate, surfactants such as polyoxyethylene sorbitan monooleate, thickening agents such as methylcellulose.

In one or more exemplary embodiments, the ocular drug delivery system is an intravitreal delivery system. For example, when the pharmaceutical composition is administered by intravitreal injection.

### Subretinal Delivery

In one or more exemplary embodiments, the ocular drug delivery system is a subretinal delivery system. Subretinal delivery, will create a depot of the substance or viral vector in close proximity to both the retinal vasculature but also the choroid vasculature.

### Retro Bulbar delivery

In one or more exemplary embodiments, the ocular drug delivery system is a retro bulbar delivery. When the target is the vasculature in the posterior eye, retrobulbar delivery is in close proximity to the arteries supplying the eye.

### Sub-tenon Delivery

In one or more exemplary embodiments, the ocular drug delivery system is a sub-tenon delivery. When the target is the vasculature in the posterior eye, the sub-tenon capsule is in close proximity to the arteries supplying the eye and could act as a reservoir for the ocular formulation.

### Sub-conjunctival Delivery

In one or more exemplary embodiments, the ocular drug delivery system is a sub-conjunctival delivery. When the target vasculature is in the more anterior part of the eye, a sub-conjunctival delivery is preferable.

In the present context, delivery and injection is used interchangeably.

### Reversal Via Viral Vectors

Transduction of genes with producing products with vasodilator potency to the retina is a possible strategy for the total prevention and treatment of retinal ischemic disorders, for example a recombinant adenovirus containing the cDNA for human prepro-CGRP. In one or more exemplary embodiments, the injection of said viral vector will transfect the subretinal area, leading to a constant production of preproCGRP and the following release of CGRP. This will lead to constant release of the vasodilator, making multiple injections unnecessary. This is a potential one-time treatment.

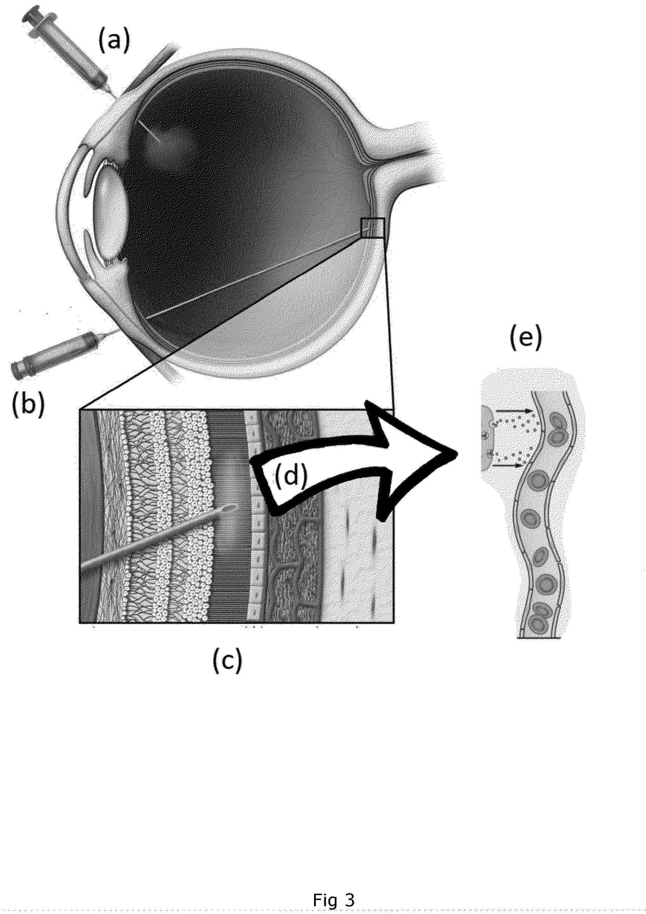

FIG.3 shows the application and mechanism of the injection of a viral vector producing CGRP leading to vasodilation. The viral vector is injected in the vitreous (a) or subretinal area (b). (c) The viral vector infects the retinal pigment epithelium, (d) the viral vector produces CGRP, and (e) CGRP dilates both the retinal and choroid vasculature.

The inventors prepares a viral vector that leads to the production of prepro-CGRP, rather than CGRP, because the precursor would facilitate the required modification and release of the active peptide from the cell.

In one or more exemplary embodiments, the ocular drug delivery system comprises the *in vivo* production of a prepropeptide, where the prepropeptide is preproCGRP or a preproCGRP derivative.

In one or more exemplary embodiments, the ocular drug delivery system is a viral vector.

### Nanotechnology based ocular drug delivery

Nanotechnology based systems with an appropriate particle size can be designed to ensure low irritation, adequate bioavailability, and ocular tissue compatibility. Several nanocarriers, such as nanoparticles, nanosuspensions, liposomes, nanomicelles and dendrimers have been developed for ocular drug delivery. Some of them have shown promising results for improving ocular bioavailability.

### Nanomicelles

Nanomicelles are the most commonly used carrier systems to formulate therapeutic agents in to clear aqueous solutions. In general, these nanomicelles are made with amphiphilic molecules. These molecules may be surfactant or polymeric in nature.

### Nanoparticles

Nanoparticles are colloidal carriers with a size range of 10 to 1000 nm. For ophthalmic delivery, nanoparticles are generally composed of lipids, proteins, natural or synthetic polymers such as albumin, sodium alginate, chitosan, poly (lactide-co-glycolide) (PLGA), polylactic acid (PLA) and polycaprolactone. Drug loaded nanoparticles can be nanocapsules or nanospheres.

Nanoparticles represent a promising candidate for ocular drug delivery because of their small size, leading to low irritation and sustained release property avoiding frequent administration. However, like aqueous solutions, nanoparticles may be eliminated rapidly from precorneal pocket. Hence, for topical administration nanoparticles with mucoadhesive properties have been developed to improve precorneal residence time. Polyethylene glycol (PEG), chitosan and hyaluronic acid are commonly employed to improve precorneal residence time of nanoparticles.

### Nanosuspensions

Nanosuspensions are colloidal dispersion of submicron drug particles stabilized by polymer(s) or surfactant(s). For ocular delivery several advantages are provided, such as sterilization, ease of eye drop formulation, less irritation, increase precorneal residence time, and enhancement in ocular bioavailability of drugs which are insoluble in tear fluid. The efficacy of nanosuspensions in improving ocular bioavailability of glucocorticoids has been demonstrated in several research studies.

### In-situ Gelling Systems

In-situ hydrogels refer to the polymeric solutions which undergo sol-gel phase transition to form viscoelastic gel in response to environmental stimuli. Gelation can be elicited by changes in temperature, pH and ions or can also be induced by UV irradiation. For ocular delivery, research studies have been more focused toward development of thermosensitive gels which respond to changes in temperature. Several thermogelling polymers have been reported for ocular delivery which includes poloxamers, multiblock copolymers made of polycaprolactone, polyethylene glycol, poly (lactide), poly (glycolide), poly (N- isopropylacrylamide) and chitosan. These thermosensitive polymers form temperature dependent micellar aggregates which gellify after a further temperature increment due to aggregation or packing. For drug delivery, these polymers are mixed with drugs in the solution state and solution can be administered. This forms an in situ gel depot at a physiological temperature. These thermosensitive gels demonstrated promising results for enhancing ocular bioavailability for both anterior and posterior segment.

### Contact Lens

Drug loaded contact lens have been developed for ocular delivery of numerous drugs such as β-blockers, antihistamines and antimicrobials. It is postulated that in presence of contact lens, drug molecules have longer residence time in the post-lens tear film which ultimately led to higher drug flux through cornea with less drug inflow into the nasolacrimal duct. Usually, the drug is loaded into contact lens by soaking the contact lenses in drug solutions. These soaked contact lenses demonstrated higher efficiency in delivering drug compared to conventional eye drops. It has been demonstrated that soft contact lenses fabricated by the molecular imprinting method have 1.6 times higher timolol loading capacity than the contact lenses prepared by a conventional method and also provided sustained timolol delivery.

### Implants

Intraocular implants are specifically designed to provide localized controlled drug release over an extended period. These devices help in circumventing multiple intraocular injections and associated complications. Usually for drug delivery to posterior ocular tissues, implants are placed intravitreally by making incision through minor surgery at pars plana which is located posterior to the lens and anterior to the retina. Though implantation is invasive procedure, these devices are gaining interest due to their associated advantages such as sustained drug release, local drug release to diseased ocular tissues in therapeutic levels, reduced side effects and ability to circumvent blood retina barrier. Several implantable devices have been developed for ocular drug delivery especially for the treatment of chronic vitreoretinal diseases.

Ocular implants are available as biodegradable and non-biodegradable drug releasing devices.

### Dendrimers

Dendrimers are characterized as nanosized, highly branched, star shaped polymeric systems. These branched polymeric systems are available in different molecular weights with terminal end amine, hydroxyl or carboxyl functional group. The terminal functional group may be utilized to conjugate targeting moieties. Dendrimers are being employed as carrier systems in drug delivery. Selection of molecular weight, size, surface charge, molecular geometry and functional group are critical to deliver drugs. The highly branched structure of dendrimers allows incorporation of a wide range of drugs, hydrophobic as well as hydrophilic. In ocular drug delivery, few promising results were reported with these branched polymeric systems.

### General

It should be understood that any feature and/or aspect discussed above in connection with the compounds according to the invention apply by analogy to the methods described herein.

### Identity

By proteins, homologues, derivatives, peptides and/or fragments thereof having an amino acid sequence at least, for example 95% identical to a reference amino acid sequence, is intended that the amino acid sequence of e.g. the peptide is identical to the reference sequence, except that the amino acid sequence may include up to 5 point mutations per each 100 amino acids of the reference amino acid sequence. In other words, to obtain a polypeptide having an amino acid sequence at least 95% identical to a reference amino acid sequence, up to 5% of the amino acids in the reference sequence may be deleted or substituted with another amino acid, or a number of amino acids up to 5% of the total amino acids in the reference sequence may be inserted into the reference sequence. These mutations of the reference sequence may occur at the amino or carboxy terminal positions of the reference amino acid sequence or anywhere between those terminal positions, interspersed either individually among amino acids in the reference sequence or in one or more contiguous groups within the reference sequence.

Methods to determine identity and similarity are codified in publicly available programs. Preferred computer program methods to determine identity and similarity between two sequences include, but are not limited to, the GCG program package (Devereux, J et al (1994)) BLASTP, BLASTN, and FASTA (Altschul, S.F. et al (1990)). The BLASTX program is publicly available from NCBI and other sources (BLAST Manual, Altschul, S.F. et al, Altschul, S.F. et al (1990)). Each sequence analysis program has a default scoring matrix and default gap penalties. In general, a molecular biologist would be expected to use the default settings established by the software program used.

The following figures and examples are provided below to illustrate the present invention. They are intended to be illustrative and are not to be construed as limiting in any way.

### SEQUENCE LISITNG

*SEQ ID NO 1: Calcitonin gene-related peptide (CGRP)* - *preproCGRP* 
MGFQKFSPFL ALSILVLLQA GSLHAAPFRS ALESSPADPA TLSEDEARLL LAALVQDYVQ

MKASELEQEQ EREGSRIIAQ KRACDTATCV THRLAGLLSR SGGVVKNNFV PTNVGSKAFG

RRRRDLQA
*SEQ ID NO 2. α-CGRP*
   **ACDTATCVTH RLAGLLSRSG GVVKNNFVPT NVGSKAF-NH2** (Disulfide bridge: 2-7)
*SEQ ID NO 3: β-CGRP*
   ACNTATCVTH RLAGLLSRSG GMVKSNFVPT NVGSKAF-NH2 (Disulfide bridge: 2-7)
*SEQ ID NO 4: preproAmylin* 
MGILKLQVFL IVLSVALNHL KATPIESHQV EKRKCNTATC ATQRLANFLV HSSNNFGAIL

SSTNVGSNTY GKRNAVEVLK REPLNYLPL
*SEQ ID NO 5: Amylin*
   **KCNTATC ATQRLANFLV HSSNNFGAIL SSTNVGSNTY** (Disulfide bridge: 2-7)
*SEQ ID NO 6: Adrenomedullin precursor* 
MKLVSVALMY LGSLAFLGAD TARLDVASEF RKKWNKWALS RGKRELRMSS SYPTGLADVK

AGPAQTLIRP QDMKGASRSP EDSSPDAARI RVKRYRQSMN NFQGLRSFGC RFGTCTVQKL

AHQIYQFTDK DKDNVAPRSK ISPQGYGRRR RRSLPEAGPG RTLVSSKPQA HGAPAPPSGS

APHFL
*SEQ ID NO 7: Adrenomedullin*
   **YRQSMN NFQGLRSFGC RFGTCTVQKL AHQIYQFTDK DKDNVAPRSK ISPQGY-NH2**
   (Disulfide bridge: 16-21)
*SEQ ID NO 8: Pramlintide*
   KCNTATCATQRLANFLV**H**SSNNFG**P**IL**PP**TNVGSNTY-NH₂
*SEQ ID NO 9: N-terminal motif 1-9*
   XCXTATCXT
*SEQ ID NO 10: Elcatonin*
   SNLSTAsuVLGKLSQELHKLQTYPRTNVGAGTP
*SEQ ID NO 11: CPRG compound*
   X₁CX₂TX₃TCX₄TX₅RLAX₆X₇LX₈RSGGX₉X₁₀X₁₁X₁₂X₁₃FVPTX₁₄VX₁₅X₁₆X₁₇X₁₈F

### BRIEF DESCRIPTION OF THE FIGURES

*Figure 1*

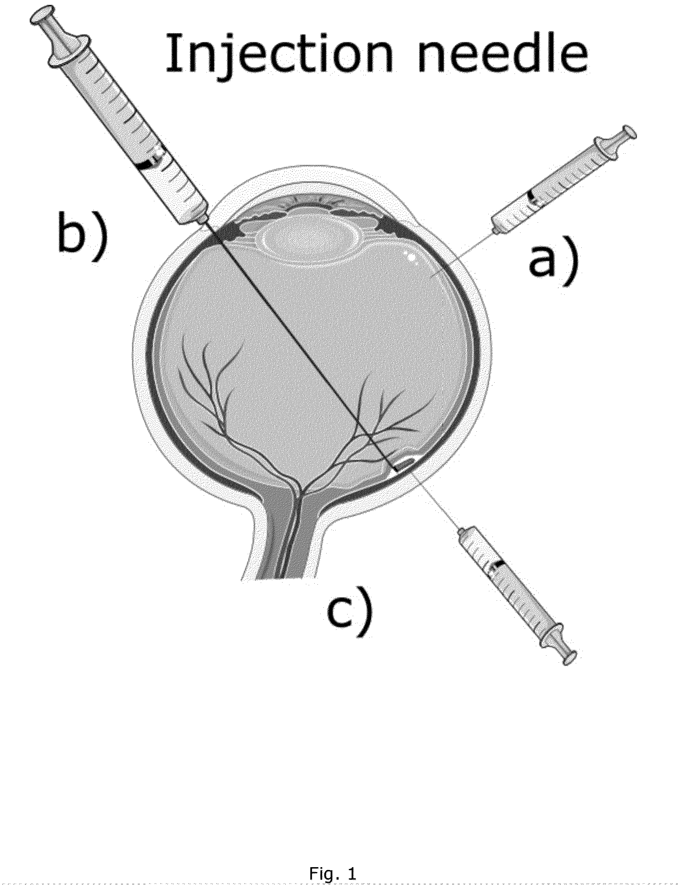

   FIG. 1 shows some of the areas of injection where the invention is applied locally in the eye. (a) Intravitreal injection (b) Internal subretinal approach with vitrectomy and subretinal injection. (c) External subretinal approach: Penetrate through sclera and choroid to reach subretinal space with or without vitrectomy.
*Figure 2*

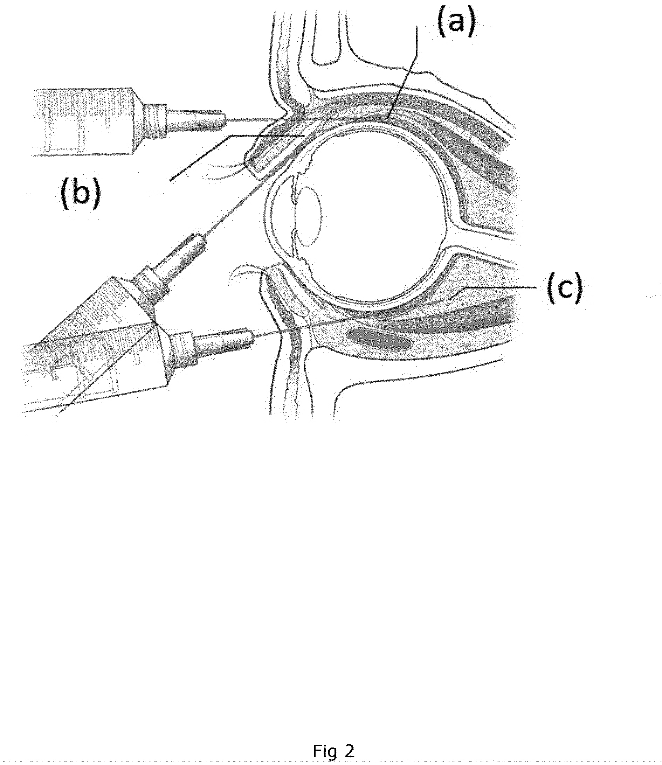

   FIG. 2 shows some of the areas of application were the invention is applied locally to the eye. (a) Sub-tenon injection (b) Sub-conjunctival (c) Retrobulbar.
*Figure 3*
   FIG.3 shows the application and mechanism of the injection of a viral vector producing CGRP leading to vasodilation. The viral vector is injected in the vitreous (a) or subretinal area (b). (c) The viral vector infects the retinal pigment epithelium (d) the viral vector produces CGRP (e) CGRP dilates both the retinal and choroid vasculature.
*Figure 4*

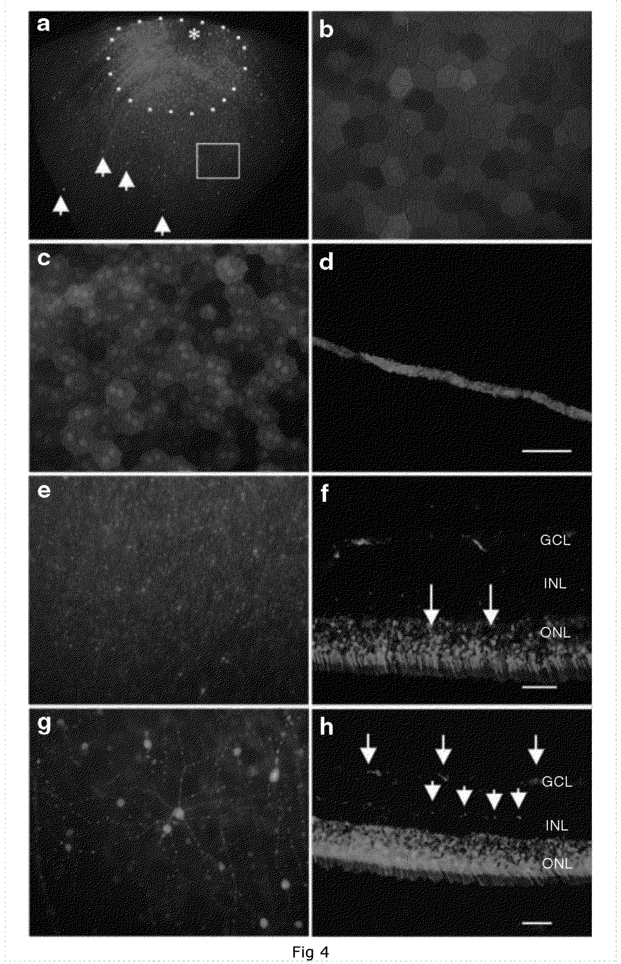

   FIG. 4 shows how a viral vector containing GFP is expressed in rat retina. GFP expression is a visual-substitute for CGRP or derivatives therefore expressed by a viral vector. (a) Subretinal injection of AAV2/8.gfp vector into rats, results in green fluorescent protein (GFP) expression in all cell-layers of the retina. (a-c,e,g,i) Retinal flatmounts and (d,f,h,j) corresponding sections of rats subretinally injected with AAV2/8.gfp. (a-c) Sclera/choroid/RPE flatmount and (d) section of the sclera/choroid/RPE flatmount showing fluorescent retinal pigment epithelium (RPE) cells. The dotted line in an outline the injected area, and the asterisk (*) marks the injection point. Arrows in a highlight GFP-expressing RPE cells outside the injected area. The square in a defines the area displayed with higher magnification in b. GFP-expressing RPE cells in c and d are located within the injected area. (e,f) Neuroretina flatmount and corresponding section display fluorescent photoreceptor cells. Arrows in f indicate photoreceptor nuclei. (g,h) Neuroretina flatmount and corresponding section show fluorescent ganglion cells and cells of the inner nuclear layer. Arrows in h indicate ganglion cells and cells of the inner nuclear layer. (i) Neuroretina flatmount displaying fluorescent axons of the ganglion cells forming the optic nerve. (j) Transverse section through the optic nerve show GFP signal in axons. Bar = 100 m. AAV, adeno-associated virus. https://doi.org/10.1038/mt.2008.41
*Figure 5*
   FIG. 5 shows the dilation of the ciliary artery when CGRP is applied topically to the eye of a Sprague dawley rat. (a) Fundus picture before CGRP is applied, the arrow points at the ciliary artery (b) fundus picture after CGRP is applied.
*Figure 6*

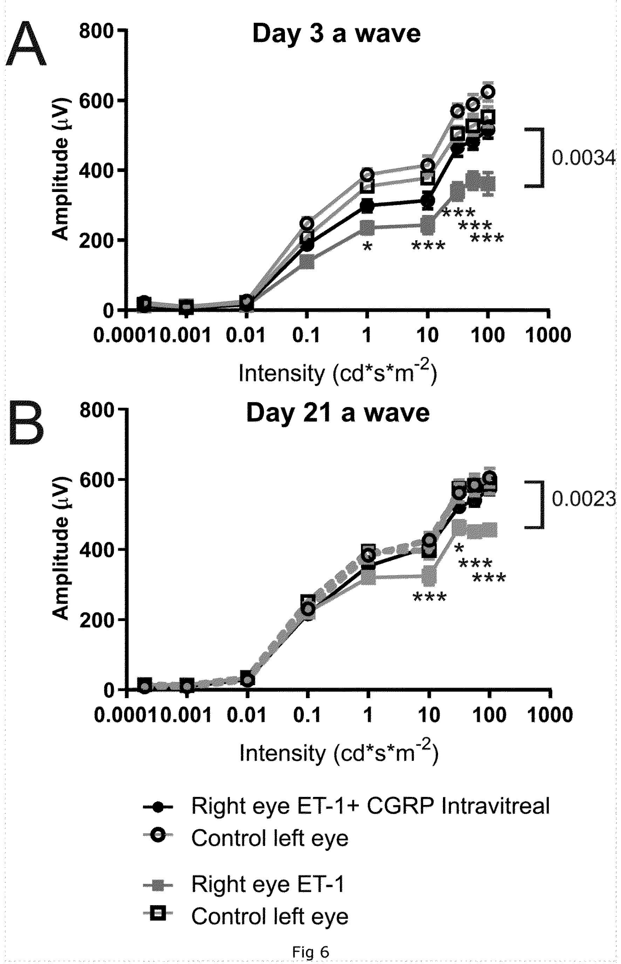

   FIG. 6 shows the Electroretinogram data after CGRP (5 µl of 200 µM, n= 11) or vehicle (PBS, 5 µl, n=9) has been applied intravitreally 10 minutes before ET-1 (5 µl of 500 µM). (a) The acute damage at day 3 in the photoreceptors, represented with the A-wave amplitude (b) the long-term damage at day 21, represented with A-wave amplitude.
*Figure 7*

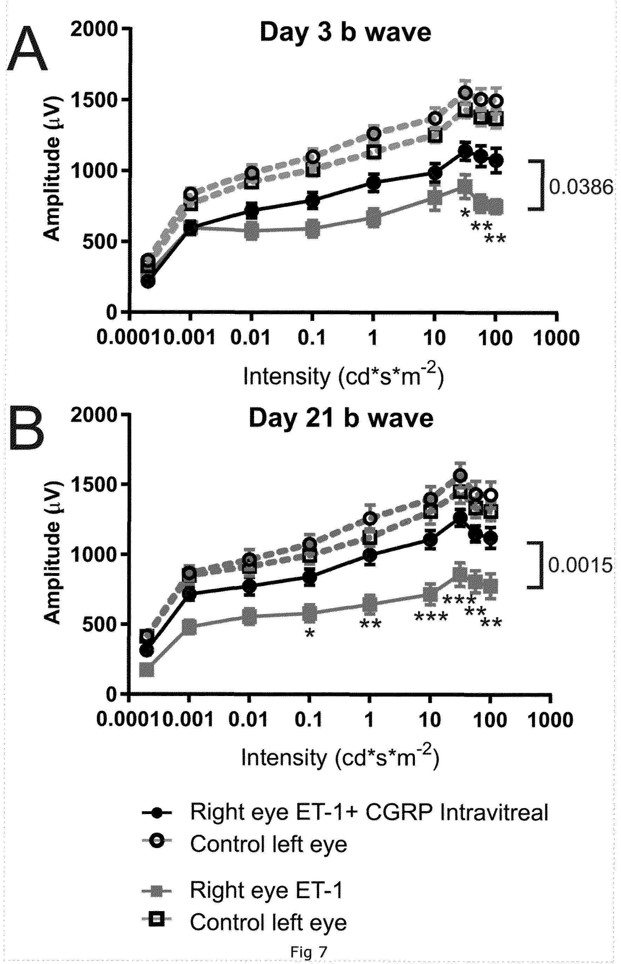

   FIG. 7 shows the Electroretinogram data after CGRP (5 µl of 200 µM, n=11) or vehicle (PBS, 5 µl, n=9) has been applied intravitreally 10 minutes before ET-1 (5 µl of 500 µM). (a) The acute damage at day 3 in the bipolar cells, represented with the B-wave amplitude (b) the long-term damage at day 21, in the bipolar cells, represented with the B-wave amplitude. Data are shown as mean ± SEM. (* P<0.05, ** P<0.01, two-way ANOVA, with Bonferroni post-test).
*Figure 8*

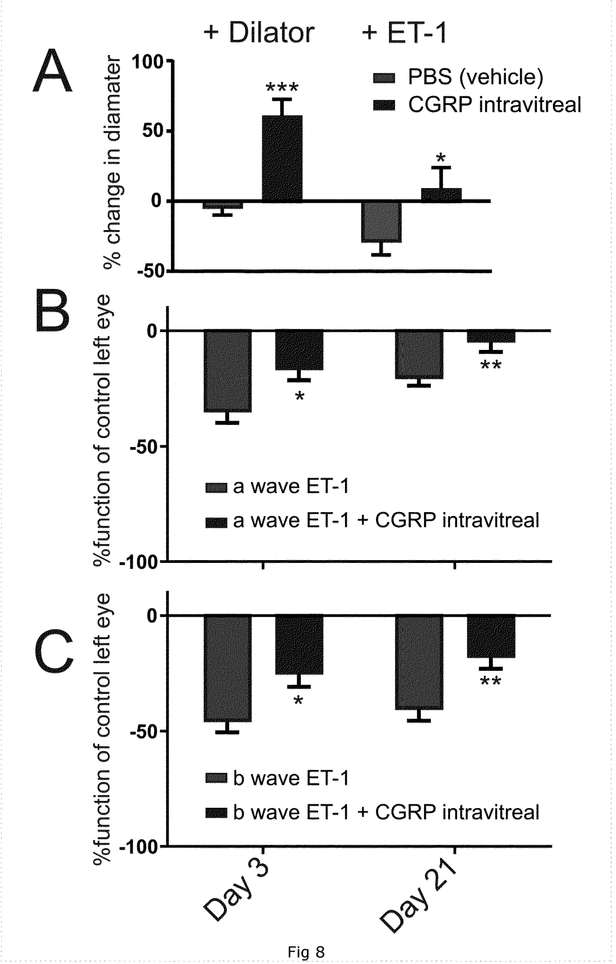

   FIG. 8 shows the dilation of the ciliary artery induced by CGRP when applied intravitreally. (a) Effect of applying the CGRP (+dilator) and the diameter of the ciliary artery after the application of 500 µM ET-1 (n=9-11). (b) Damage of the retina represented as function loss of photoreceptors cells (A-wave amplitudes) compared to the un-injected eye (n= 9-11). (c) Damage of the retina represented as function loss of bipolar cells (B-wave amplitudes) compared to the un-injected eye. Data is shown as mean ± SEM. (* P<0.05, ** P<0.01 ; Student's T-test).
*Figure 9*

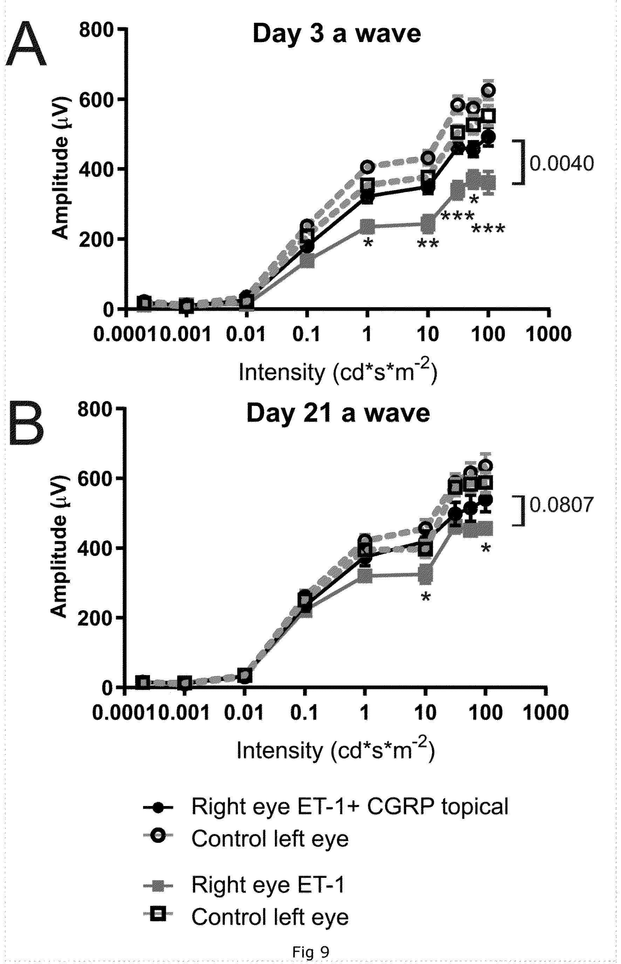

   FIG. 9 shows the Electroretinogram data after topical CGRP (10 µl of 200 µM, n=8) has been applied topically 10 minutes before intravitreal ET-1 (5 µl of 500 µM). (a) The acute damage at day 3 in the photoreceptors, represented with the A-wave amplitude (b) the long-term damage at day 21, represented with the A-wave amplitude. Data is shown as mean ± SEM. (* P<0.05, ** P<0.01, two-way ANOVA, with Bonferroni post-test).
*Figure 10*

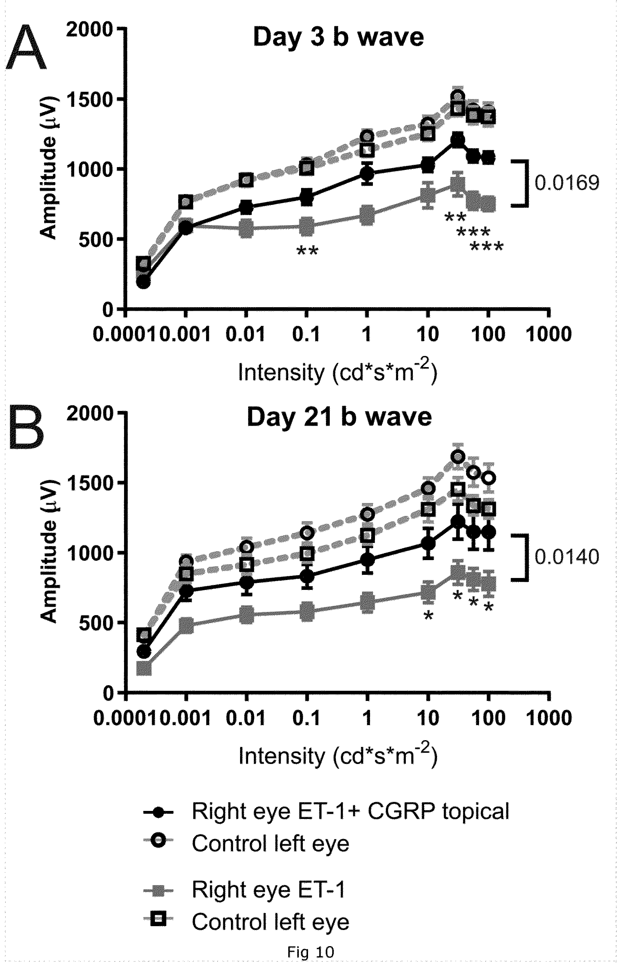

   FIG. 10 shows the Electroretinogram data after topical CGRP (10 µl of 200 µM, n=8) has been applied topically 10 minutes before intravitreal ET-1 (5 µl of 500 µM) (a) The acute damage at day 3 in the bipolar cells, represented with the B-wave amplitude (b) the long term damage at day 21, in the bipolar cells, represented with the B-wave amplitude. Data is shown as mean ± SEM. (* P<0.05, ** P<0.01, *** P<0.001 , two-way ANOVA, with Bonferroni post-test).
*Figure 11*

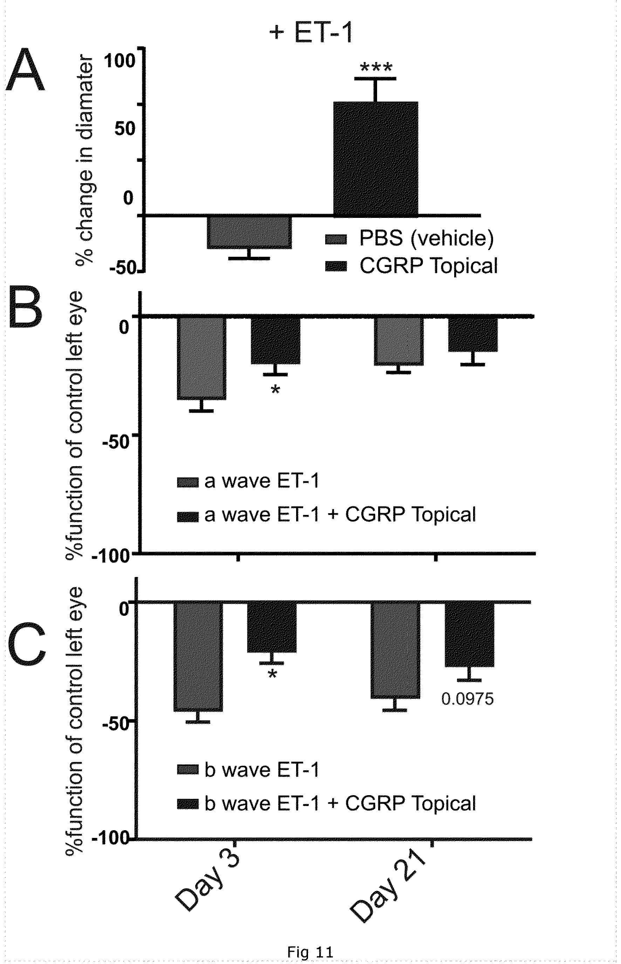

   FIG. 11 shows the dilation of the ciliary artery induced by CGRP when applied topically. (a) Effect of applying the CGRP (+dilator) after the application of 500 µM) ET-1. (b) Damage of the retina represented as function loss of photoreceptors cells (A-wave amplitudes) compared to the un-injected eye. (c) Damage of the retina represented as function loss of bipolar cells (B-wave amplitudes) compared to the un-injected eye. Data is shown as mean ± SEM. (* P<0.05, ** P<0.01, *** P<0.001; Student's T-test).
*Figure 12*

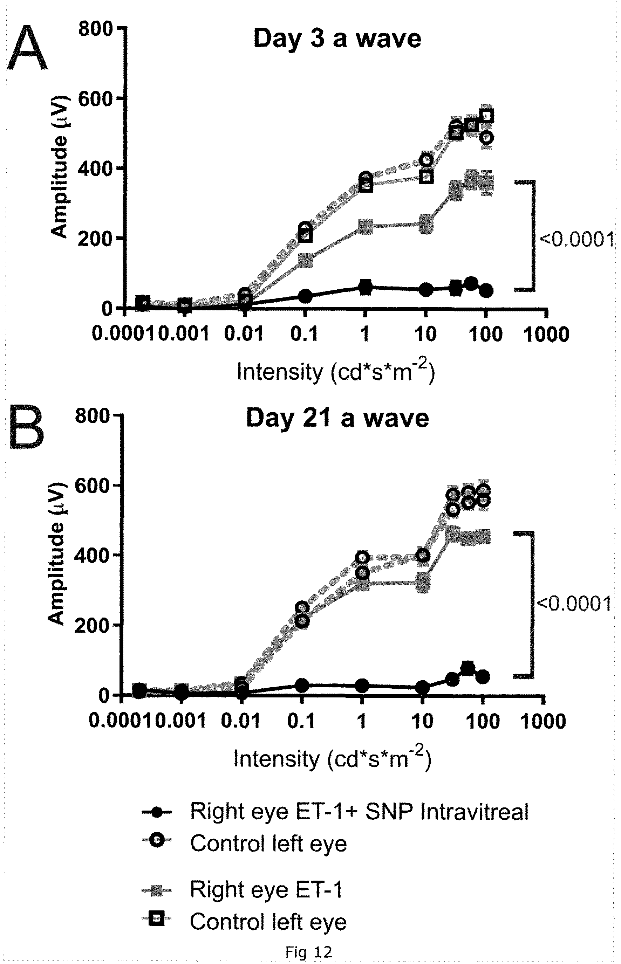

   FIG. 12 shows the Electroretinogram data after sodium nitroprusside (5 µl of 0.5 mM, n=6) or vehicle (PBS, 5 µl, n=9) has been applied intravitreally 10 minutes before ET-1 (5 µl of 500 µM). (a) the acute damage at day 3 in the photoreceptors, represented with the A-wave amplitude (b) the long term damage at day 21, represented with the A-wave amplitude. Data is shown as mean ± SEM. (* P<0.05, ** P<0.01, *** P<0.001 two-way ANOVA, with Bonferroni post-test).
*Figure 13*

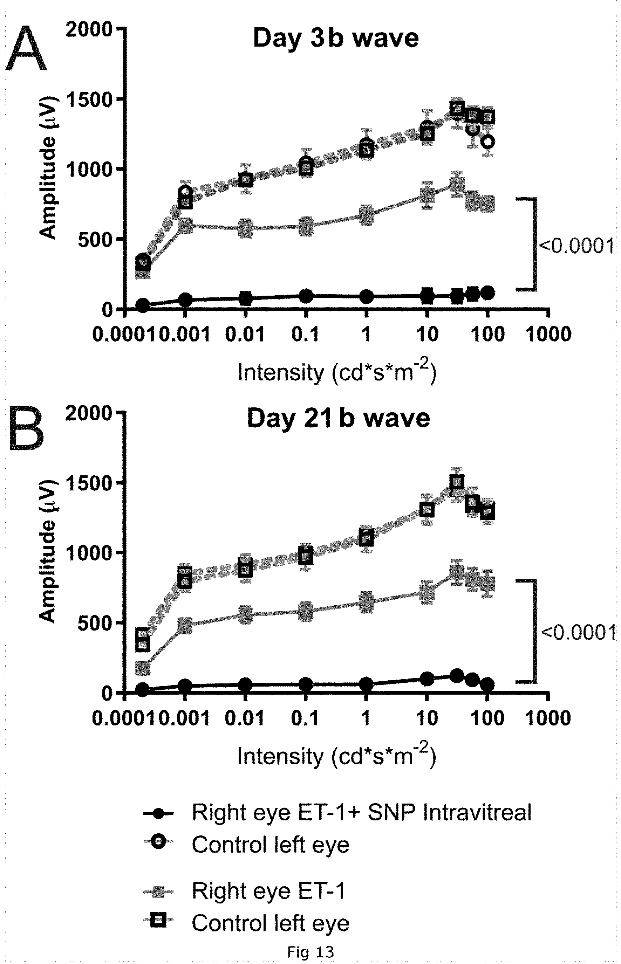

   FIG. 13 shows the Electroretinogram data after sodium nitroprusside (5 µl of 0.5 mM, n=8) or vehicle (PBS, 5 µl, n=9) has been applied intravitreally 10 minutes before ET-1 (5 µl of 500 µM). (a) The acute damage at day 3 in the bipolar cells, represented with the B-wave amplitude (n=6-9) (b) the long term damage at day 21, in the bipolar cells, represented with the B-wave amplitude (n=6-9). Data is shown as mean ± SEM. (* P<0.05, ** P<0.01, *** P<0.001 , two-way ANOVA, with Bonferroni post-test).
*Figure 14*

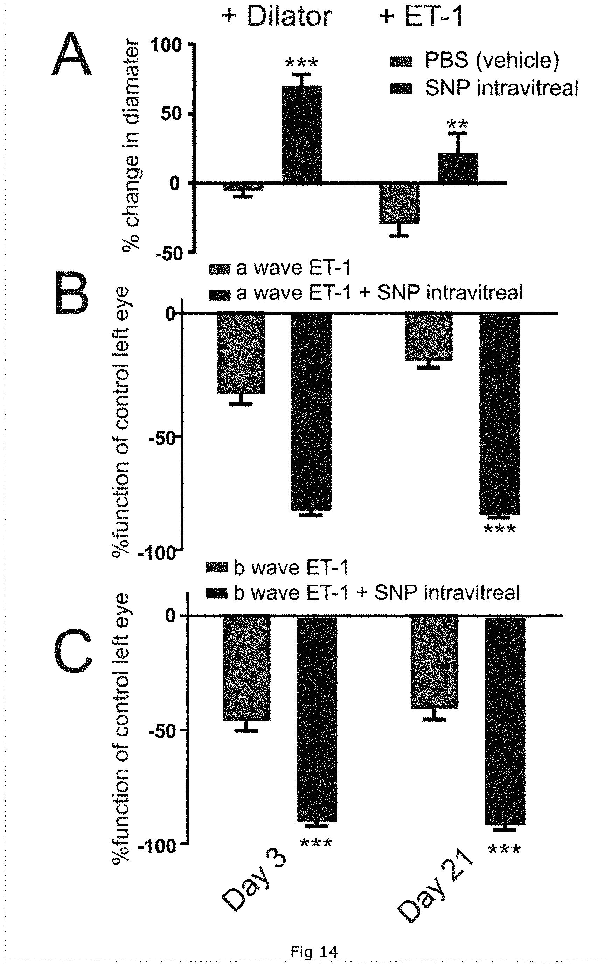

   FIG. 14 shows the dilation of the ciliary artery induced by sodium nitroprusside (5 µl of 0.5 mM, n=6) when applied intravitreally. (a) Effect of applying the sodium nitroprusside (5 µl of 0.5 mM, +dilator) and the diameter of the ciliary artery after the application of 500 µM ET-1. (b) Damage of the retina represented as function loss of photoreceptors cells (A-wave amplitudes) compared to the un-injected eye (n=6-9). (c) Damage of the retina represented as function loss of bipolar cells (B-wave amplitudes) compared to the un-injected eye (n=6-9). Data is shown as mean ± SEM. (* P<0.05, ** P<0.01, *** P<0.001; Student's T-test).

### EXAMPLES

### Example 1 - The vasodilator effect in Endothelin-1 induced retinal ischemia's

### Background

Retinal diseases such as glaucoma, diabetic retinopathy, age-related macular degeneration and retinitis pigmentosa are the major causes of blindness. A large number of experimental animal models using rodents (rats and mice) have been used for the evaluations of the pathogenesis and novel therapeutic candidates in retinal diseases.

Endothelin-1 has been implicated in the pathogenesis of ischemic diseases such as glaucoma. Glaucoma is one of the leading causes of blindness, affecting approximately 66 million patients world-wide. Previous studies have shown that endothelin-1 levels were elevated in glaucomatous eyes of humans. Previous studies with ET-1 intravitreal administration have found that it leads to retinal ganglion cell (RGC) loss, caspase-3 activation, retinal vessel vasoconstriction, electroretinography deficits, RGC and retinal nerve fiber layer (RNFL) thickness changes within one week of intravitreal injection.

### Methods

### Intravitreal injections.

Rats were anesthetized with 90mg/kg ketamine & 10mg/kg xylazine (i.p). Oxybuprocain 0.4% Anesthetic was applied topically to the eyes.

Guided under a stereoscopic microscope to avoid lens and retinal injury, 5 µl intravitreal injection of 500uM ET-1 (Phoenix Pharmaceuticals, Inc.) was performed using a digital controller and UltraMicroPump III, with NanoFil 10 µl syringe (WPI) connected to intraoucular injector with a 34g bevelled needle.

Injection was performed through the sclera, inserted approximately 1 mm posterior to the corneal limbus at a 45°angle to avoid contact with the lens capsule and direct the contents, 1.5mm into the vitreous chamber, in the right eye (FIG.1). The needle remained in the vitreous cavity for 30 seconds after injection and was then gently withdrawn. A cotton swab was then placed on the injection site to prevent withdrawal of injected solution, and the eye ball was gently massaged to help with the distribution of compound through the vitreous. Antibiotic ointment was applied in the conjunctival sac after the intravitreal injections. The contralateral eye was used as the control eye.

Fundus imaging was performed to document any changes to the retina or vasculature. Animals recover on the heating plate and returned to the stables once awake and resuming normal behavior. The rats are tested according to the following schedule.

Day 0 - ET-1 is administered intravitreally alone or in combination with the current invention. Fundus is recorded before and after injection.
Day 3 - Animals are anesthetized to perform ERG Measures
Day 21 - Animals are anesthetized to perform ERG Measures

### Treatment regimes

A) intravitreal 5µl PBS (vehicle) 10 min before ET-1
B) intravitreal 5 µl CGRP (200µM) 10 min before ET-1
C) intravitreal 5 µl SNP (500µM) 10 min before ET-1
D) topical application 10 µl CGRP (200µM) before ET-1

### ERG

After overnight dark adaptation for at least 12 hours, the animals were anesthetized with 90 mg/kg ketamine and 10 mg/kg xylazine delivered by a single intraperitoneal injection. Then, oxybuprocaine anesthetic was applied topically to the eyes and the pupils dilated with Mydriacyl and Metaoxedrin under dim red-light illumination. The body temperature was maintained with a heating pad set to 37°C. Using a Ganzfeld stimulator, white light flashes (0.0002-100 cd-s/m2) were produced under scotopic conditions. ERGs were recorded from both eyes simultaneously with a gold wire electrode loops, placed on the corneal apex, with a drop of saline applied to the corneal surface for hydration during the testing procedure. A reference electrode was placed in the mouth and a ground electrode inserted sub dermally in the tail of the animal. The scotopic A-wave amplitude and B-wave amplitude were recorded and evaluated.

### Results

In order to evaluate the functional effect of ET-1 on the retina, ERG was performed at 3 and 21 days post injection. Focus was put on the damage at day 21, the long-term outcome. At day 21, photoreceptor activity, represented by the A-wave amplitude, showed a significant functional deficit when comparing ET-1 induced ischemic right eyes (5µL 500µM, n = 9) and the control left eyes (n = 9). The control left eye had an avg. max A-wave amplitude of 582 ± 70 µV compared to 457 ± 40 µV in the ET-1 induced ischemia right eye at day 21. The photoreceptor function deficit was less at day 21, suggesting a partial recovery, still with functional deficit in the ET-1 induced ischemia right eye (FIG. 6). The control left eye had an avg. max B-wave amplitude of 1368 ± 209 µV compared to 804 ± 188 µV in the ET-1 induced ischemia right eye at day 21. The photoreceptor function deficit was slightly improved at day 21, suggesting a partial recovery, still with strong functional deficit in the ET-1 induced ischemia right eye (FIG. 8).

In relation to retinal function, adding CGRP intravitreally (5µL 200µM, n=11) or topical (10µL, 200 µM), n=8) had strong protective effect. Firstly, they both caused a dilation of the ciliary artery (FIG.7 and FIG. 11). Intravitreal CGRP dilated the ciliary artery to 61 ± 12 % increase from baseline, before the injection of ET-1. Topical CGRP also increased the diameter (102 ± 21 % increase from baseline), visualized after the injection of intravitreal ET-1. After applying ET-1, the ciliary artery of the eye pretreated with intravitreal CGRP, had a diameter of 9 ±15 % above baseline, compared to ET-1 plus vehicle where there was a contraction (-29 ±9% from baseline).

Applying CGRP reduced the ET-1 induced damage greatly at day 21, with the average A-wave amplitude when applied intravitreally being 545 ± 45 µV or applied topically being 511 ± 100 µV compared to the ET-1 ischemic eye (457 ± 40 µV), seen in FIG. 6 and FIG. 9. Similarly, CGRP reduced the ET-1 induced damage on the bipolar cells at day 21, with the B-wave amplitude when applied intravitally being 1181 ± 202 µV) or applied topically being 1088 ± 411 µV compared to the ET-1 ischemic right eye (804 ± 188 µV), seen in FIG. 7 and FIG. 10.

Sodium nitroprusside (SNP) is a well-known vasodilator. When SNP was applied intravitreally (5 µL 500µM, n=6) it caused a vasodilation of the ciliary artery by 70 ± 9 % increase from baseline (FIG 14). Similar to that of intravitreal CGRP injection (61 ±12 % increase from baseline). Following the ET-1 injection, the dilation was maintained (21 ± 14 % over baseline) and the diameter was larger than that of ET-1 alone (-29 ± 9 % from baseline) and similar to the CGRP group (9 ±15 % over baseline). However, applying SNP completely destroyed retinal function observed at day 21, with an average A-wave amplitude when applied intravitreally (61 ± 26 µV) compared to the ET-1 control eye (457 ± 40 µV), as seen in FIG. 12. Since there are no A-wave amplitude, no B-wave amplitude was expected, and that was indeed the case, as the B-wave amplitudes when SNP was applied intravitally was (91 ± 86 µV) compared to the ET-1 right eye (804 ± 188 µY), as seen in FIG. 13.

Interestingly there was an increase in a and B-wave amplitude function after applying topical CGRP. Suggesting that the left eye is slightly potentiated. This has been seen before with a potential cross talk between the eyes. Due to this the A/B-wave amplitude receiver appear smaller (FIG. 11) although the absolute improvement when applying CGRP intravitreally or topically is nearly identical.

Combined, these data show that the addition of CGRP greatly preventing loss of retinal function. This is surprising as a well characterized and potent vasodilator SNP had the opposite effect, as the photoreceptor damage was further enhanced.

### Example 2 - Subretinal Injection of rAAV Vectors

Subretinal injection of an rAAV vector can achieve efficient transduction of RPE and other retinal cells because subretinal injection induces a bleb of concentrated virus in intimate contact with RPE cells and the neural retina (FIG. 3). In addition, the subretinal space has a relatively high degree of immunoprivilege and typically very little evidence of inflammation is seen in the vicinity of the injection site. Thus, subretinal injection is a preferred route for delivery of rAAV vectors in mammalian retina. However, other routes of delivery may be used, for example, intravitreal injection.

The subretinal injection is performed either in both eyes or unilaterally in the right eye. All procedures are performed under aseptic conditions, using sterile reagents, syringes and appropriate personal protection equipment. The eye is examined and the success of the subretinal injection is confirmed by visualization of a bleb containing fluorescein. The success of injection and the degree of retinal damage (hemorrhage) are scored.

To study the rAAV vector-induced gene transduction and cell-type specifics in the retina, the eGFP expression in retinal cross sections and RPE/retina flatmounts are examined. One approach used to identify the eGFP expressing cell types is to co-label eGFP positive cells with retinal cell markers by immunocytochemistry staining in cryosections (FIG. 4). Furthermore, production of CGRP in the vitreous and subretinal space is measured in an CGRP specific ELISA kit conforming functional transfection.

Further the outcome on ET-1 induced ischemia either acutely or long term, is tested using the electroretinogram as described in Example 1.

### Example 3 - Neuroprotection

Since retinal ischemia is not limited to the lack of flow but also implies that the retina of the eye is involved in the disease or disorder. The retina comprises neurons, and as such retinal disorders as such are disorders in which neuritogenesis and/or neuroprotection are desirable. The application of intravitreal CGRP, topical application of CGRP or production of CGRP from a viral vector, is combined with the intravitreal injection of a neuroprotectant, for example Glial cell-line derived neurotrophic factor (GDNF). This further enhances the retinal function as flow is restored following the CGRP application/production and neurons/retina is further protected by the neuroprotectant. This is shown with further increase in the A and B-wave amplitudes, compared to CGRP alone.

## Claims

1. A vasodilator for use in reversing the effects of vasoconstriction in the retina in a mammal, wherein the vasodilator acts via the calcitonin receptor-like receptor (CRLR/RAMP-1) and wherein the vasodilator has a peptide motif in the N-terminal end having at least 99% identity to SEQ ID NO: 9 and a threonine (T) in amino acid position 6, and has at least 75% amino acid sequence similarity with SEQ ID NO: 2.

2. A vasodilator for use according to claim 1, wherein Cys2 and Cys7 are cyclised with a disulfide bond.

3. A vasodilator for use according to any of claims 1-2, wherein the vasodilator reversing the effects of vasoconstriction in the retina for both the photoreceptors (A-wave amplitude) and the Muller and ON-bipolar cells (B-wave amplitude) by at least 10%, when measured by Electroretinography in the mammal compared to the ET-1 induced ischemia alone, at day 3 and at day 21 after the vasodilator is first applied to said mammal.

4. A vasodilator for use according to any of claims 1-3, wherein the vasodilator targets the ocular vasculature, the vasodilator targets the retinal vasculature, the smooth muscle cells (SMC), pericytes and/or the endothelia cells.

5. A vasodilator for use according to any of claims 1-4, wherein the vasoconstriction of the retina causes glaucoma or diabetic retinopathy.

6. A vasodilator for use according to any of claims 1-4, wherein said use is treatment of glaucoma.

7. A composition comprising a vasodilator for use according to any of claims 1-6.

8. An ocular drug delivery system comprising a vasodilator for use or a composition for use according to any of claims 1-7.

9. An ocular drug delivery system according to claim 8, wherein the ocular drug delivery system is a viral vector or a topical eye drop delivery system.

## Patentansprüche

1. Ein Vasodilatans zur Verwendung bei der Umkehrung der Auswirkungen der Vasokonstriktion in der Netzhaut bei einem Säugetier, wobei das Vasodilatans über den Calcitonin-Rezeptorähnlichen Rezeptor (CRLR/RAMP-1) wirkt und wobei das Vasodilatans ein Peptidmotiv im N-terminalen Ende mit mindestens 99% Identität mit der SEQ ID Nr.: 9 und einem Threonin (T) in der Aminosäureposition 6, sowie mindestens 75% Aminosäuresequenzähnlichkeit mit der SEQ ID Nr: 2 aufweist.

2. Ein Vasodilatans zur Verwendung nach Anspruch 1, wobei das Cys2 und das Cys7 mit einer Disulfidbindung cyclisiert sind.

3. Ein Vasodilatans zur Verwendung nach einem der Ansprüche 1 bis 2, wobei das Vasodilatans die Auswirkungen der Vasokonstriktion in der Netzhaut sowohl für die Photorezeptoren (A-Wellenamplitude) als auch für die Müller- und die ON-Bipolarzellen (B-Wellenamplitude) um mindestens 10 %, gemessen durch Elektroretinographie bei dem Säugetier im Vergleich zu der ET-1-induzierten Ischämie allein, am Tag 3 und am Tag 21 nach der ersten Anwendung des Vasodilatans bei dem Säugetier, umkehrt.

4. Ein Vasodilatans zur Verwendung nach einem der Ansprüche 1 bis 3, wobei das Vasodilatans auf das Augengefäßsystem abzielt, das Vasodilatans auf das Netzhautgefäßsystem, die glatten Muskelzellen (SMC), die Perizyten und/oder die Endothelienzellen abzielt.

5. Ein Vasodilatans zur Verwendung nach einem der Ansprüche 1 bis 4, wobei die Vasokonstriktion der Netzhaut ein Glaukom oder eine diabetische Retinopathie verursacht.

6. Ein Vasodilatans zur Verwendung nach einem der Ansprüche 1 bis 4, wobei die Verwendung die Behandlung von Glaukom ist.

7. Eine Zusammensetzung umfassend ein Vasodilatans zur Verwendung nach einem der Ansprüche 1 bis 6.

8. Ein okuläres Arzneimittelabgabesystem umfassend ein Vasodilatans zur Verwendung oder eine Zusammensetzung zur Verwendung nach einem der Ansprüche 1 bis 7.

9. Ein okuläres Arzneimittelabgabesystem nach Anspruch 8, wobei das okuläre Arzneimittelabgabesystem ein viraler Vektor oder ein topisches Augentropfenabgabesystem ist.

## Revendications

1. Un vasodilatateur destiné à être utilisé pour inverser les effets de la vasoconstriction dans la rétine chez un mammifère, dans lequel le vasodilatateur agit via le récepteur de type récepteur de la calcitonine (CRLR/RAMP-1) et dans lequel le vasodilatateur a un motif peptidique à l'extrémité N-terminale ayant au moins 99 % d'identité avec la SEQ ID No : 9 et une thréonine (T) en position d'acide aminé 6, et a au moins 75 % de similarité de séquence d'acides aminés avec la SEQ ID No : 2.

2. Un vasodilatateur pour l'utilisation selon la revendication 1, dans lequel la Cys2 et la Cys7 sont cyclisées avec une liaison disulfure.

3. Un vasodilatateur pour l'utilisation selon l'une quelconque des revendications 1 à 2, dans lequel le vasodilatateur inverse les effets de la vasoconstriction dans la rétine à la fois pour les photorécepteurs (amplitude de l'onde A) et pour les cellules de Müller et ON-bipolaires (amplitude de l'onde B) d'au moins 10 %, mesuré par électrorétinographie chez le mammifère par rapport à l'ischémie induite par l'ET-1 seule, au jour 3 et au jour 21 après la première application du vasodilatateur sur ledit mammifère.

4. Un vasodilatateur pour l'utilisation selon l'une quelconque des revendications 1 à 3, dans lequel le vasodilatateur cible le système vasculaire oculaire, le vasodilatateur cible le système vasculaire rétinien, les cellules musculaires lisses (SMC), les péricytes et/ou les cellules endothéliales.

5. Un vasodilatateur pour l'utilisation selon l'une quelconque des revendications 1 à 4, dans lequel la vasoconstriction de la rétine provoque un glaucome ou une rétinopathie diabétique.

6. Un vasodilatateur pour l'utilisation selon l'une quelconque des revendications 1 à 4, dans lequel ladite utilisation est le traitement du glaucome.

7. Une composition comprenant un vasodilatateur pour l'utilisation selon l'une quelconque des revendications 1 à 6.

8. Un système d'administration de médicament oculaire comprenant un vasodilatateur pour l'utilisation ou une composition pour l'utilisation selon l'une quelconque des revendications 1 à 7.

9. Un système d'administration de médicament oculaire selon la revendication 8, dans lequel le système d'administration de médicament oculaire est un vecteur viral ou un système d'administration topique de gouttes oculaires.
